# EUROPEAN PATENT APPLICATION

(11) **EP 2 966 068 A1**
(43) Date of publication of application: **13.01.2016**
(21) Application number: 14182478.9
(22) Date of filing: 27.08.2014
(51) Int. Cl.: C07D 295/04, C07C 317/04, C07C 321/14, C07D 295/215, A61K 9/127

(54) **Synthesis and use of amino lipids**

(30) Priority: 08.07.2014 EP 14176142
(71) Applicant: Incella GmbH, 76344 Eggenstein-Leopoldshafen (DE); Karlsruher Institut für Technologie, 76131 Karlsruhe (DE)
(72) Inventor: Li, Linxian, 76344 Eggenstein-Leopoldshafen (DE); Shankara, Girish Karadka, 76351 Karlsruhe (DE); Du, Xing, 76344 Eggenstein-Leopoldshafen (DE); Wu, Yihang, 76131 Karlsruhe (DE); Davidson, Gary, 76676 Graben- Neudorf (DE); Levkin, Pavel, 76344 Eggenstein- Leopoldshafen (DE)
(74) Representative: Hoppe, Georg Johannes

(57) **Abstract**

The present invention provides novel amino lipids and a method for synthesizing these compounds.

## Description

The present invention pertains to amino lipids and their synthesis. Such (cationic) amino lipids have good properties as transfection agents. They can be used to produce lipid particles, especially liposomes, allowing the delivery of bioactive agents into cells. The simplicity of the synthesis allows the development of a combinatorial library of (cationic) amino lipids in a kit-like manner. The compounds contained in this library can be screened for particular properties, in particular for the transfection of various cell lines. The invention also refers to the use of lipid particles containing the neutral or cationic amino lipids as medicament.

### Background

Of the various reagents used to transfect cells with bioactive agents such as nucleic acids, those based on liposome mediated delivery are widely acknowledged to be the most effective. This is due mostly to their efficiency and ease of use. Liposomes are artificially prepared spherical vesicles made of a lipid bilayer. To deliver the molecules to sites of action, the lipid bilayer can fuse with other bilayers such as the cell membrane, thus delivering the liposome contents inside the cell.

Liposomes are used for drug delivery due to their unique properties. A liposome encapsulates a region of aqueous solution inside a hydrophobic membrane; dissolved hydrophilic solutes cannot readily pass through the lipids. Hydrophobic chemicals can be dissolved into the membrane, and in this way liposome can carry both hydrophobic molecules and hydrophilic molecules. Liposomes can be combined with bioactive agents such as drugs, nucleic acids, peptides etc. and used to deliver these agents for the regulation of a cells biochemical pathway. This opens possibilities for new treatments of diseases.

For delivery of negatively charged nucleic acid, cationic lipids are the most effective transfection agents. Cationic lipids represent a promising class of synthetic materials for DNA delivery. To date, there are several commercialized cationic lipids but the number of cationic lipids for safe and effective delivery of genes is still limited.

In "Cationic liposomes for gene therapy" (Miller AD, Angew Chem Int Ed. 1998, 37: 1768-1785) most of the commonly used and commercially available transfection agents are described. However, conventional lipid synthesis typically requires individually optimized, multiple-step synthesis, including time-intensive procedures such as chemical protection and deprotection, use and removal of catalysts, solvent exchanges and purification.

Cationic lipids need to be combined with natural phospholipids (referred to as helper lipids) to form liposomes that can be more efficiently incorporated into cell membranes. By combining liposomes with DNA or drugs, which alone cannot diffuse through the membrane of the target cell, they can be (indiscriminately) delivered past the lipid bilayer. The use of liposomes for transformation or transfection of DNA into a host cell is known as lipofection.

Although liposomal reagents represent the state of the art with respect to cell transfection agents, they have the following drawbacks: Firstly, many cell lines (such as primary cells) cannot be effectively transfected at the moment, even with liposomal reagents. Secondly, they are relatively difficult and expensive to synthesize, often resulting in a high price.

As a consequence of the second point, many laboratories use less efficient, cheaper alternatives for transfection (e.g. calcium phosphate). There is a concrete requirement for new transfection agents that are easy to synthesize and which have good transfection yields for a wide variety of cell types. As an alternative, it would be helpful to dispose an easy combinatorial synthesis of transfection agents allowing the production of a variety of different compounds.

Accordingly, it was an object of the present invention to provide novel (cationic) amino lipids and a method for their synthesis. The method should be generic, economic and easy to perform. This generic method allows the development of a library of cationic amino lipids. Generation of such lipid libraries (containing of hundreds of different lipid molecules) greatly enhances the identification of lipids harboring optimal transfection reagent properties.

It was a further object of the present invention to provide lipid particles, especially liposomes, containing said (cationic) amino lipids. In particular, these lipid particles or liposomes should be able to deliver bioactive agents through cell membranes.

Another object was the use of said lipid particles or liposomes for the treatment of diseases.

### Description of the invention

The invention provides novel amino lipids with the following general formula (I): 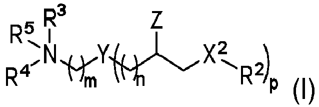 wherein Z is either hydrogen or -X¹R¹,
R¹ and R² are the same or different and independently from each other C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either 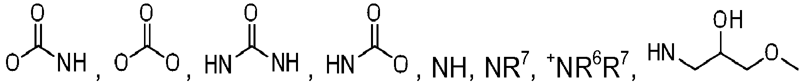 or heterocycles of the formula 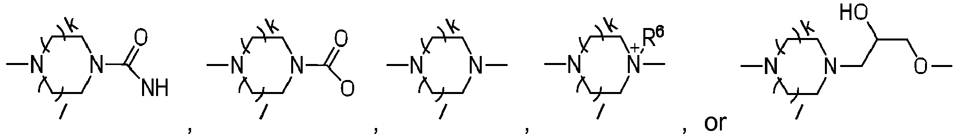 wherein k and l are integers from 0 to 2.

R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ and R⁶ are either absent or is hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein in one embodiment, Y = N for p = 2 or 3.

In one embodiment of the invention R³ and R⁴ are the same or different alkyl amines.

In a preferred embodiment of the invention, R¹ and R² are the same or different and independently C₆-C₂₄ alkyl, more preferred, R¹ and R² are the same C₆-C₁₈ alkyl.

In one embodiment the invention relates to amino lipids of formula (Ib) 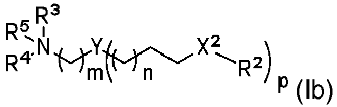 wherein the variables are defined as above.

In another embodiment, X¹ and X² are the same or different, either S=O or S(=O)₂.

In one embodiment, the present invention provides amino lipids of the general formula (II): 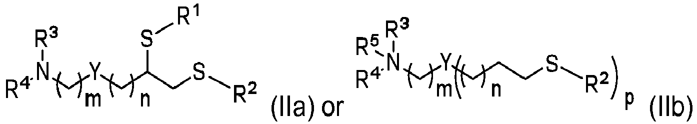 R¹ and R² are the same C₆-C₁₈ alkyl
Y is either 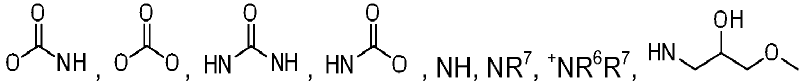 or heterocycles of the formula 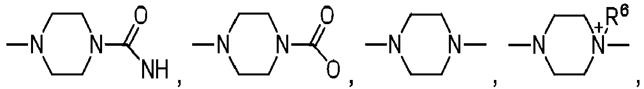 or 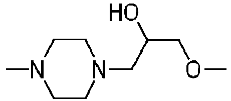 R³ and R⁴ are either the same or different C₁-C₁₂ alkyls, wherein alkyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ and R⁶ are either absent or is hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein in one embodiment, Y = N for p = 2 or 3.

In a preferred embodiment of the compounds according to the formulas (I) or (II), n and m are independently of each other an integer of 1, 2 or 3, and p is independently an integer of 1 or 2.

Particularly preferred embodiments correspond to the structures of formula (IIIa), (IIIb), (IIIc), (IIId), (IIIe), (IIIf), (IIIg), (IIIh), (IIIi), (IIIj), (IIIk), (IIIl), (IIIm), (IIIn), (IIIo), (IIIp), (IIIq), (IIIr), (Ills), (IIIt), (IIIu), (IIIv), or (IIIw) 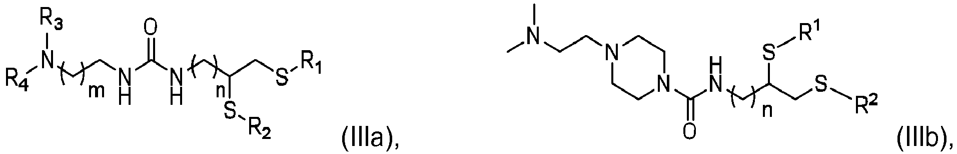 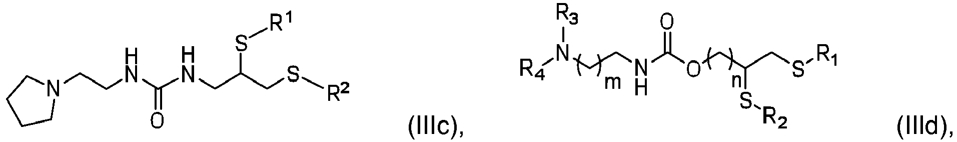 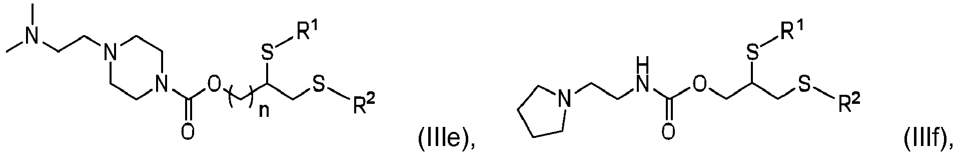 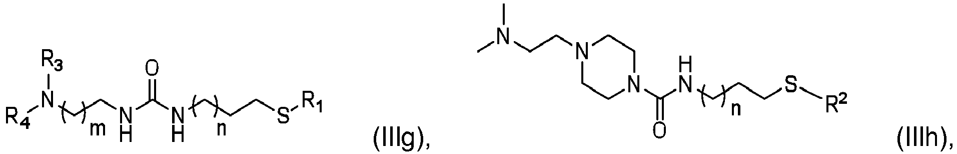 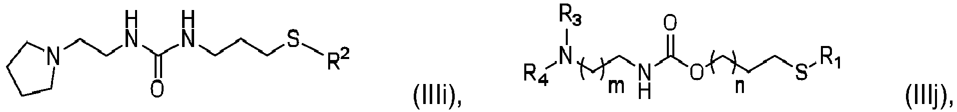 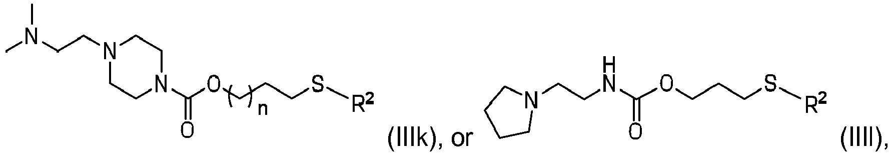 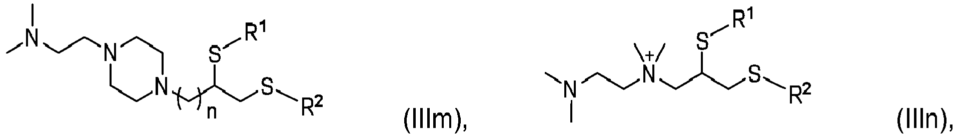 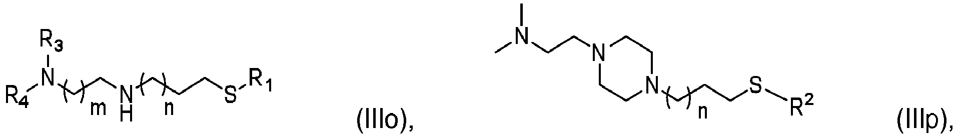 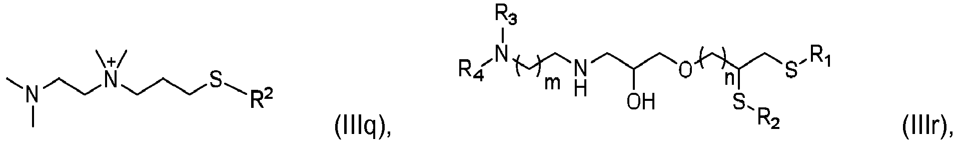 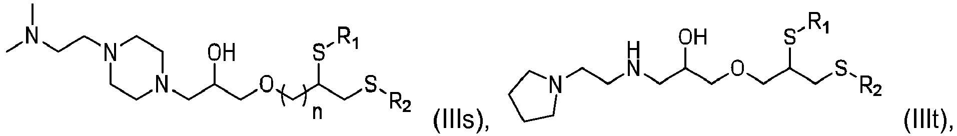 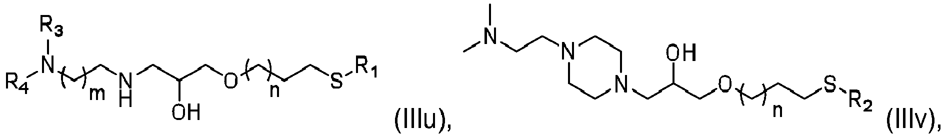 or 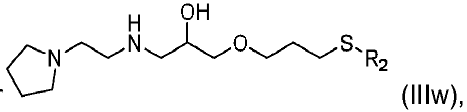 wherein R¹ and R² are the same C₁₁-C₁₂ alkyls,
R³ and R⁴ are the same C₁-C₂ alkyls,
m is an integer from 1 to 2, n is an integer from 1 to 3.

In one particularly preferred embodiment of the invention, in formula I and II Y is either 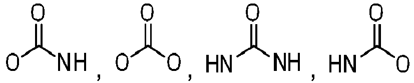 or heterocycles of the formula 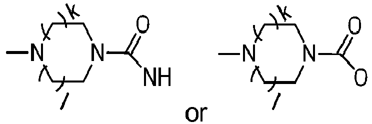 wherein k and l are integers from 0 to 2,
particularly preferably Y is either 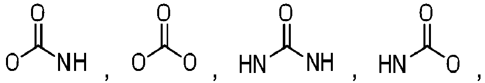 or
heterocycles of the formula 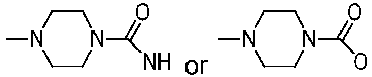 and most preferably the amino lipids according to the invention comprise a structure according to IIIa to IIIl.

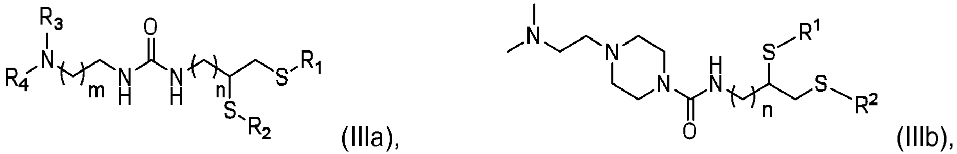

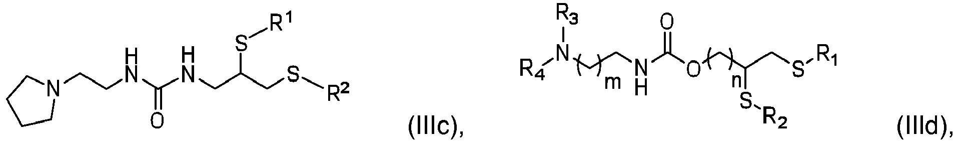

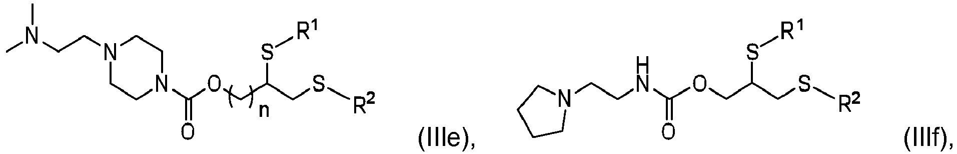

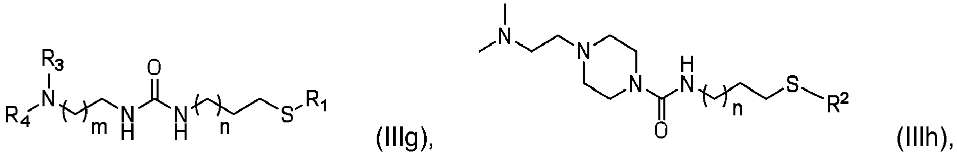

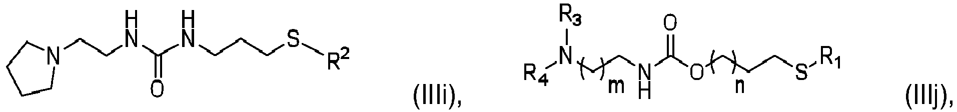

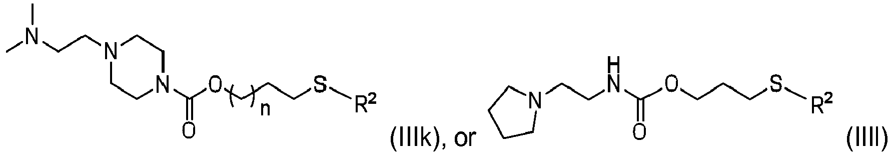

In another particularly preferred embodiment, in formula I and II Y is either NH, NR⁷, ⁺NR⁶R⁷, 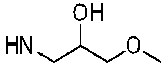 or heterocycles of the formula 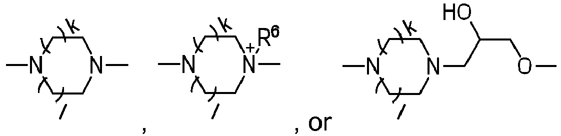 wherein k and l are integers from 0 to 2, R⁶ is either absent or hydrogen or C₁-C₁₂ alkyls to provide a quaternary amine, R⁷ is either hydrogen or C₁-C₁₂ alkyl, particularly preferably Y is either NH, NR⁷, ⁺NR⁶R⁷, 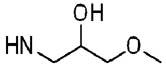 or heterocycles of the formula 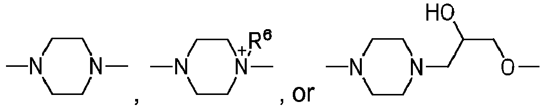 and most preferably the amino lipids according to the invention comprise a structure according to IIIm to IIIw. 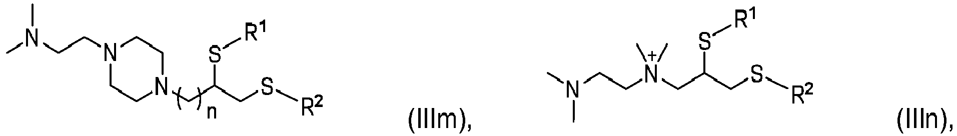 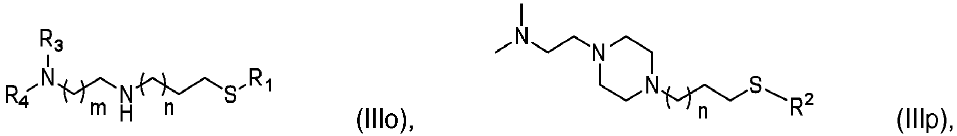 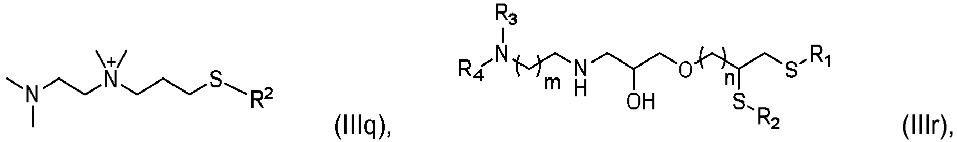 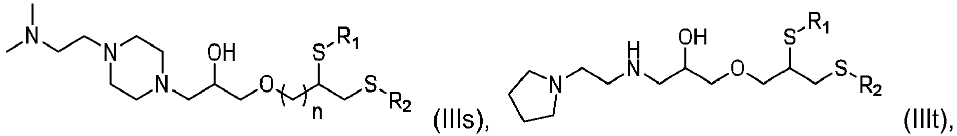 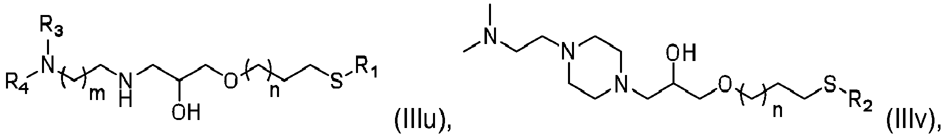 or

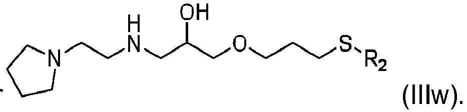

The invention also provides novel amino lipids with the following general formula (Ic): 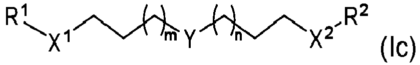 wherein R¹ and R² are the same or different and independently from each other C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either 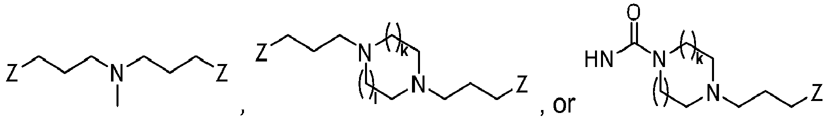 wherein Z is 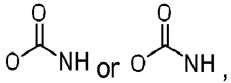 k and l are integers from 0 to 2.
m is an integer from 1 to 12 and n is an integer from 1 to 12.

### Synthesis methods

The present invention also provides a method to synthesize amino lipids according to formula I and Ic, preferably Ib. The method represents the first parallel synthesis of large libraries of ionizable (cationic) amino lipids based on thiol-yne chemistry in liquid-phase combinatorial synthesis without chromatography purification.

The method according to the invention comprises the step of the reaction of alkynes or alkenes of the general formula (VI) 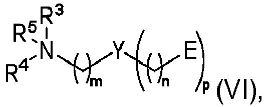 with the compounds of the general formula, HS-R¹ and HS-R²,
under either UV-irradiation or using a radical initiator, to yield a compound of the general formula (VII) 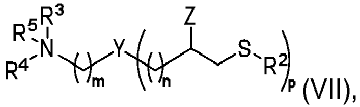 wherein E is either CH=CH₂ or C=CH,
Z is either hydrogen or -S-R¹,
R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
Y is either 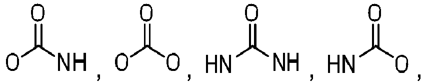 NH, NR⁷, ⁺NR⁶R⁷, 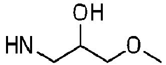 or heterocycles of the formula 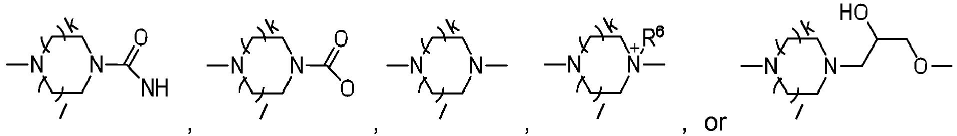 wherein k and l are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ and R⁶ are either absent or are hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein in one embodiment Y = N for p = 2 or 3.

In one embodiment R³ and R⁴ are the same; in another embodiment, R³ and R⁴ are different alkyl amines.

This reaction is conducted via a radical mechanism. Chemical radical sources can be added to start the reaction or it can be simply conducted under sunlight. In a preferred embodiment the reaction is started by UV irradiation.

In one particularly preferred embodiment of the invention the method further comprises the step of
oxidation of the thioethers of general formula (VII) into sulfoxide (S=O) and/or sulfone (S(=O)₂) using an oxidation agent to synthesize a sulfoxide or sulfone group containing amino lipid.

The oxidation of the thioethers is preferably performed at room temperature with aqueous hydrogen peroxide in a solvent such as methanol. In another embodiment of the invention this oxidation of the thioethers can be catalyzed by a catalyst such as titanium-containing zeolites. This reaction has already been proposed by Hulea et al. (Hulea V, Moreau P, Renzo FD. Thioether oxidation by hydrogen peroxide using titanium-containing zeolites as catalysts. Journal of Molecular Catalysis A: Chemcial. 1996, 111:325-332).

In one preferred embodiment the method according to the invention further comprises the following two steps:
a) reaction of alkynes or alkenes of the general formula (IVa), (IVb), (IVc), or (IVd) 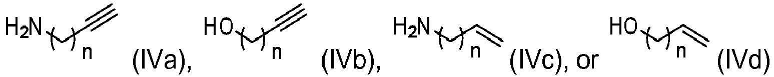 wherein n is an integer from 1 to 12,
   with the compound 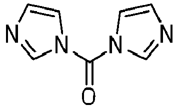 to yield a compound of the general formula (Va), (Vb), (Vc), or (Vd) 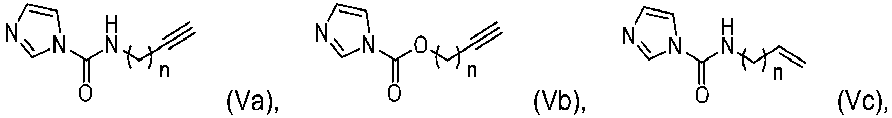 or 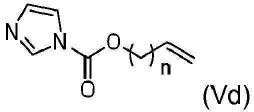
b) reaction of alkynes or alkenes of the general formula (Va), (Vb), (Vc), or (Vd) 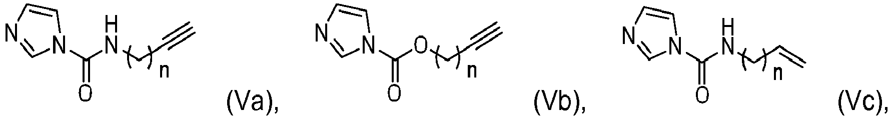 or 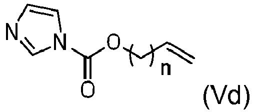 with an amine of the general formula (R³R⁴R⁵N)(CH₂)ₘZ, with m as an integer from 1 to 12, wherein Z is OH, NH₂, NH, or a secondary heterocyclic amine of the formula 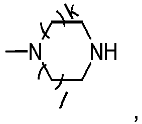 wherein k and l are integers from 0 to 2,
   to yield a compound of the general formula (VI') 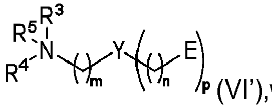 ,wherein E is either CH=CH₂ or C=CH,
   R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
   Y is either 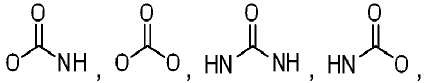 or heterocycles of the formula 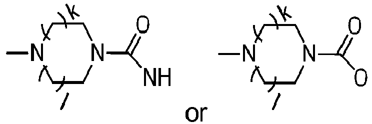 or wherein k and l are integers from 0 to 2,
   R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
   R⁵ is either absent or is hydrogen or C₁-C₁₂ alkyl to provide a quaternary amine,
   m is an integer from 1 to 12, n is an integer from 1 to 12, and p is an integer from 1 to 3, wherein in one embodiment Y = N for p = 2 or 3.

In one embodiment R³ and R⁴ are the same; in another embodiment, R³ and R⁴ are different alkyl amines.

In an alternative preferred embodiment the method according to the invention comprises the following step:
reaction of alkynes or alkenes of the general formula (IVe), (IVf), (IVg), or (IVh) 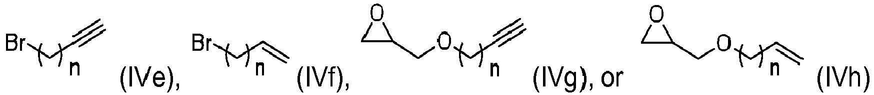 wherein n is an integer from 1 to 12,
with an amine of the general formula (R³R⁴R⁵N)(CH₂)ₘZ, with m as an integer from 1 to 12, wherein Z is NH₂, NH, N or a secondary heterocyclic amine of the formula 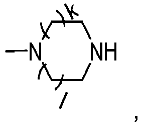 wherein k and l are integers from 0 to 2, to yield a compound of the general formula (VI") 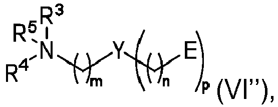 wherein E is either CH=CH₂ or C=CH,
R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
Y is either NH, NR⁷, ⁺NR⁶R⁷, 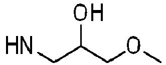 or heterocycles of the formula 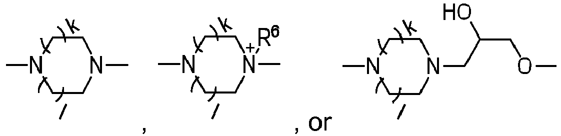 wherein k and l are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ and R⁶ are either absent or are hydrogens or C₁-C₁₂ alkyls to provide a quaternary amine,
R⁷ is either hydrogen or C₁-C₁₂ alkyl,
m is an integer from 1 to 12, n is an integer from 1 to 12, and p is an integer from 1 to 3, wherein in one embodiment Y = N for p =2 or 3.

In one embodiment R³ and R⁴ are the same; in another embodiment, R³ and R⁴ are different alkyl amines.

In one embodiment, the invention relates to a method to synthesize amino lipids according to formula I, 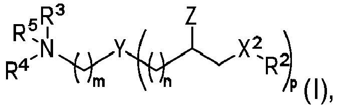 wherein Z is either hydrogen or -X¹-R¹,
R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either 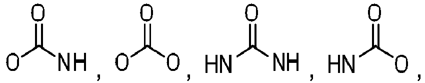 or heterocycles of the formula 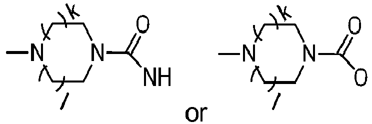 or wherein k and l are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ is either absent or is hydrogen or C₁-C₁₂ alkyl to provide a quaternary amine,
m is an integer from 1 to 12, n is an integer from 1 to 12, and p is an integer from 1 to 3.

In one embodiment R³ and R⁴ are the same; in another embodiment, R³ and R⁴ are different alkyl amines.

The method represents the first parallel synthesis of large libraries of ionisable cationic amino lipids based on thiol-yne chemistry in liquid-phase combinatorial synthesis (without chromatography purification) comprising the following steps:

The first step is reaction of alkynes or alkenes of the general formula (IVa), (IVb), (IVc), or (IVd) 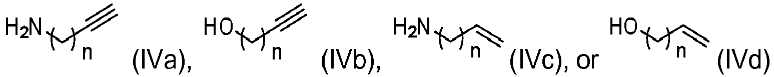 wherein n is an integer from 1 to 12,
with the compound 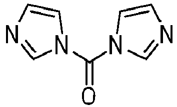 to yield a compound of the general formula (Va), (Vb), (Vc), or (Vd) 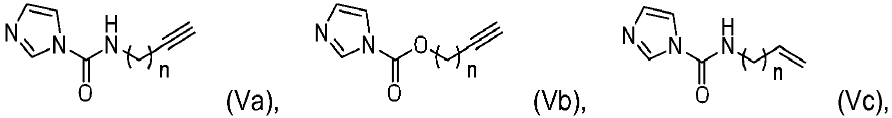 or

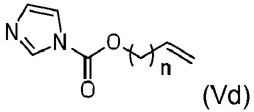

The second step is a reaction of alkynes or alkenes of the general formula (Va), (Vb), (Vc), or (Vd) 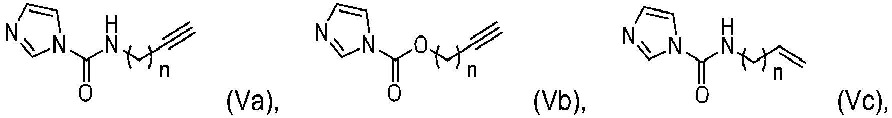 or 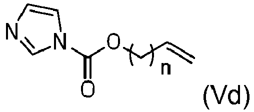 with an amine of the general formula (R³R⁴R⁵N)(CH₂)ₘZ, with m as an integer from 1 to 12, wherein Z is OH, NH₂, NH, or a secondary heterocyclic amine of the formula 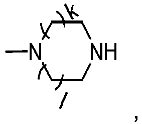 wherein k and l are integers from 0 to 2,
to yield a compound of the general formula (VI') 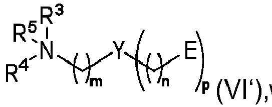 wherein n, m, p, Y, E, R³, R⁴ and R⁵ are defined as above.

The third step is the reaction of alkynes of the general formula (VI) with the compounds of the general formula, HS-R¹ and HS-R²,
wherein R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group, preferably under either UV-irradiation or using a radical initiator, to yield a compound of the general formula (VII') 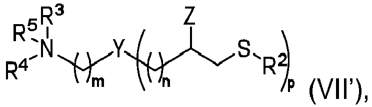 wherein n, m, p, Y, Z, R¹, R², R³, R⁴ and R⁵ are defined as above.

This reaction is conducted preferably via a radical mechanism. Chemical radical sources can be added to start the reaction or it can be simply conducted under sunlight. In a preferred embodiment the reaction is started by UV irradiation.

The optional fourth step is an oxidation reaction of the thioether groups of the product of second step with an oxidizing reagent to yield a compound of the general formula (I), wherein n, m, p, Y, R¹, R², R³, R⁴ and R⁵ are defined as above and X¹, X² are the same or different, either S=O or S(=O)₂. The fourth step is preferably performed at room temperature with aqueous hydrogen peroxide in a solvent such as methanol.

The fourth step can further been catalyzed by a catalyst such as titanium-containing zeolites. This reaction has already been proposed by Hulea et al. (Hulea V, Moreau P, Renzo FD. Thioether oxidation by hydrogen peroxide using titanium-containing zeolites as catalysts. Journal of Molecular Catalysis A: Chemcial. 1996, 111:325-332). Surprisingly this reaction does not affect other functional groups of the amino lipids of the second step.

In one embodiment of the invention, this fourth step is mandatory.

In another embodiment, the invention relates to a method to synthesize amino lipids according to formula (I), 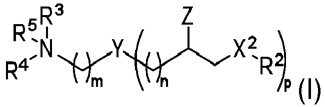 wherein Z is either hydrogen or -X¹-R¹,
R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either NH, NR⁷, ⁺NR⁶R⁷, 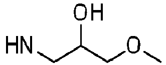 or heterocycles of the formula 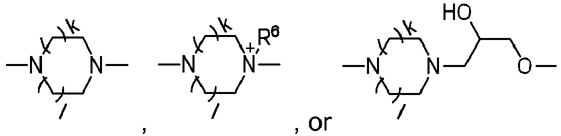 wherein k and l are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen.
R⁵ and R⁶ are either absent or are hydrogen or C₁-C₁₂ alkyls to provide a quaternary amine,
R⁷ is either hydrogen or C₁-C₁₂ alkyl,
m is an integer from 1 to 12 and n is an integer from 1 to 12 and p is an integer from 1 to 3, wherein in one embodiment Y = N for p =2 or 3.

In one embodiment R³ and R⁴ are the same; in another embodiment, R³ and R⁴ are different alkyl amines.

The method represents the first parallel synthesis of large libraries of ionizable cationic amino lipids based on thiol-yne chemistry in liquid-phase combinatorial synthesis without chromatography purification comprising the following steps:
The first step is reaction of alkynes or alkenes of the general formula (IVe), (IVf), (IVg), or (IVh) 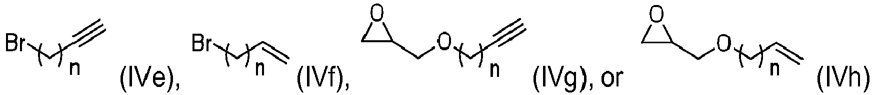 wherein n is an integer from 1 to 12,
with an amine of the general formula (R³R⁴R⁵N)(CH₂)ₘZ, with m as an integer from 1 to 12, wherein Z is NH₂, NH, N or a secondary heterocyclic amine of the formula 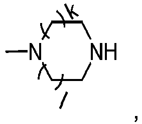 wherein k and l are integers from 0 to 2, to yield a compound of the general formula (VI") 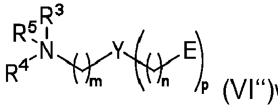 wherein E is either CH=CH₂ or C=CH,
Y is either NH, NR⁷, ⁺NR⁶R⁷, 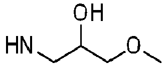 or heterocycles of the formula 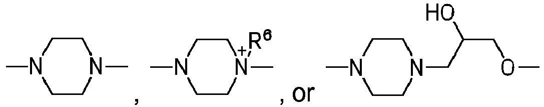 R³ and R⁴ are either the same or different C₁-C₁₂ alkyls, wherein alkyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ and R⁶ are either absent or are hydrogen or C₁-C₁₂ alkyls to provide a quaternary amine,
R⁷ is either hydrogen or C₁-C₁₂ alkyl,
m is an integer from 1 to 12 and n is an integer from 1 to 12 and p is an integer from 1 to 3. In a preferred embodiment n and m are independently an integer of 1, 2 or 3, and p is independently an integer of 1 or 2.

In one embodiment R³ and R⁴ are the same; in another embodiment, R³ and R⁴ are different alkyl amines.

The second step is reaction of alkynes or alkenes of the general formula (VI") with the compounds of the general formula, HS-R¹ and HS-R², wherein R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group, under either UV-irradiation or using a radical initiator, to yield a compound of the general formula (VII") 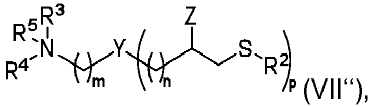 wherein n, m, p, Y, R¹, R², R³, R⁴ and R⁵ are defined as above for formula VI".

This reaction is conducted via a radical mechanism. Chemical radical sources can be added to start the reaction or it can be simply conducted under sunlight. In a preferred embodiment the reaction is started by UV irradiation.

The optional third step is an oxidation reaction of the thioether groups of the product of second step with an oxidising reagent to yield a compound of the general formula (I), wherein n, m, p, Y, Z, R¹, R², R³, R⁴ and R⁵ are defined as above and X¹, X² are the same or different, either S=O or S(=O)₂. The third step is preferably performed at room temperature with aqueous hydrogen peroxide in a solvent such as methanol. The third step can further been catalyzed by a catalyst such as titanium-containing zeolites. This reaction has already been proposed by Hulea et al. (Hulea V, Moreau P, Renzo FD. Thioether oxidation by hydrogen peroxide using titanium-containing zeolites as catalysts. Journal of Molecular Catalysis A: Chemcial. 1996, 111:325-332). Surprisingly this reaction does not affect other functional groups of the amino lipids of the second step.

In one embodiment, this third step is mandatory.

In one embodiment the invention comprises the reaction alkynes or alkenes of the general formula (VIII) 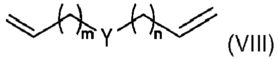 with the compounds of the general formula, HS-R¹ and HS-R²,
under either UV-irradiation or using a radical initiator, to yield a compound of the general formula (Ic) 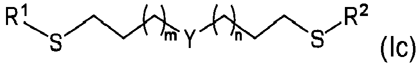 Wherein R¹ and R² are the same or different and independently from each other C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either 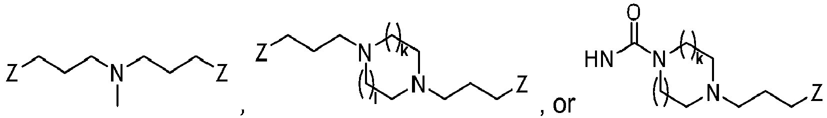 wherein Z is 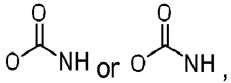 k and l are integers from 0 to 2.
m is an integer from 1 to 12 and n is an integer from 1 to 12.

In one particularly preferred embodiment of the invention the method further comprises the step of oxidation of the thioethers of general formula (Ic) into sulfoxide (S=O) and/or sulfone (S(=O)₂) using an oxidation agent to synthesize a sulfoxide or sulfone group containing amino lipid.

The oxidation of the thioethers is preferably performed at room temperature with aqueous hydrogen peroxide in a solvent such as methanol. In another embodiment of the invention this oxidation of the thioethers can be catalyzed by a catalyst such as titanium-containing zeolites. This reaction has already been proposed by Hulea et al. (Hulea V, Moreau P, Renzo FD. Thioether oxidation by hydrogen peroxide using titanium-containing zeolites as catalysts. Journal of Molecular Catalysis A: Chemcial. 1996, 111:325-332).

These methods according to the invention are very versatile; they can be used to synthesize large libraries of (cationic) amino lipids for rapid cell-based screening assay in a very inexpensive manner. The resulting compounds all have both a hydrophobic character due to their long non-polar residues and a hydrophilic character due to their amino group. This amphiphilic character can be used to form lipid particles, e.g. lipid bilayers, micelles, liposomes, etc. Moreover, the amino group of these compounds confers a cationic charge, which is useful for transfection agents. This library of different compounds with new characteristics can be tested easily for their transfection capacity of a wide variety of cell types.

### Lipid particles

Another embodiment of the present invention is directed to lipid particles containing an amino lipid according to formulas I to III. Within the scope of the invention, the term 'lipid particle' means nanosized objects made of amino lipids which are placed into an aqueous solution. These particles are inter alia lipid bilayer vesicles (liposomes), a multi-lamellar vesicles or miscelles.

In a preferred embodiment of the present invention, said nanoparticles are liposomes containing an amino lipid according to formulas I to III. Within the scope of the invention liposomes are microvesicles composed of a bilayer of lipid amphipathic (amphiphilic) molecules enclosing an aqueous compartment.

Liposome formation is not a spontaneous process. Lipid vesicles are formed first when lipids such as phospholipids are placed in water and consequently form one bilayer or a series of bilayers, each separated by water molecules. Liposomes can be created by sonicating lipid vesicles in water.

Within the scope of the invention, the term 'lipid bilayer' means a thin membrane made of two layers of lipid molecules.

The term 'micelle' means an aggregate of surfactant molecules dispersed in a liquid colloid. A typical micelle in aqueous solution forms an aggregate with the hydrophilic head regions in contact with water, sequestering the hydrophobic single tail region in the micelle center.

Within the scope of the invention, the term 'cells' means a generic term and encompass the cultivation of individual cells, tissues, organs, insect cells, avian cells, fish cells, amphibian cells, mammalian cells, primary cells, continuous cell lines, stem cells and/or genetically engineered cells, such as recombinant cells expressing a hetereologous polypeptide or protein. Recombinant cells include, for example, cells expressing heterologous polypeptides or proteins, such as a growth factor or a blood factor.

In one embodiment, said lipid particles or liposomes further contain a helper lipid. Such a helper lipid is a non-cationic lipid, preferably a non-cationic phospholipid. Liposomes consisting of a mixture of cationic amino lipids and non-cationic (neutral) phospholipids are the most effective for nucleic acid delivery into cells. In an even more preferred embodiment said non-cationic lipid is DOPE. In a most preferred embodiment, helper lipid and amino lipid of claims 1-4 are added in a 1:1 ratio to form liposomes.

In a further embodiment, the lipid particle or liposome further comprises a sterol. Sterol, like cholesterol, is a natural component in cell membranes. It can be used to stabilize the particle, and help the integration into a cell membrane.

In another embodiment of the invention, the lipid particles or liposomes further contain a bioactive agent. Within the scope of this invention a bioactive agent is one which has a biological effect when introduced into a cell or host, for example by stimulating an immune response or an inflammatory response, by exerting enzymatic activity or by complementing a mutation, etc. bioactive agents include inter alia nucleic acids, peptides, proteins, antibodies and small molecules.

When a liposome is used to encapsulate a drug substance either within the lipid bilayer or in the interior aqueous space of the liposome the term 'liposome drug' can be employed.

In a most preferred embodiment, the bioactive agent is a nucleic acid. In another preferred embodiment said bioactive agent is a member optionally selected from the group consisting of: an antineoplastic agent, an antibiotic, an immunomodulator, an anti-inflammatory agent, an agent acting on the central nervous system, a polypeptide or a polypeptoid.

In yet another embodiment the lipid particle or liposome further contains at least one polyethylene glycol (PEG)-lipid. PEG lipids help to protect the particles and their cargo from degradation in-vivo. Moreover, PEG form a protective layer over the liposome surface and increase the circulating time in vivo. It can be used in liposome drug delivery (PEG-liposome).

Lipid particles or liposomes containing a bioactive agent can be used to deliver any of a variety of therapeutic agents into cells. The present invention encompasses the use of lipid particles, especially liposomes, as described above for delivering a bioactive agent into a cell.

Preferably said bioactive agent is a nucleic acid, including but not limited to, RNA, antisense oligonucleotide, a DNA, a plasmid, a ribosomal RNA (rRNA), a micro RNA (miRNA), transfer RNA (tRNA), a small inhibitory RNA (siRNA) or small nuclear RNA (snRNA). The bioactive agent may also be an antineoplastic agent, an antibiotic, an immunomodulator, an anti-inflammatory agent, an agent acting on the central nervous system, antigens or fragments thereof, proteins, peptides, polypeptoid, vaccines and small-molecules or mixtures thereof.

As has been set out above, lipid particles or liposomes containing amino lipids as defined in the present invention are suitable to deliver bioactive agents into cells. The wide variety of different amino lipids which can be synthesized by the mentioned versatile synthesis method can be screened for particular characteristics that are conferred to the liposomes. Important characteristics are for example transfection efficiency, cytotoxicity, adhesion of the agent to be delivered into the cell, stability of the liposomes, size of the liposomes, etc. The present method allows the creation of specifically adapted liposomes for particular applications.

For example, lipid particles (liposomes) can be used for transfecting multicellular tissues or organisms. This offers the possibility of a novel therapeutic treatment of patients.

According to the present invention, a patient can be any mammal, preferably selected from the group consisting of human, mouse, rat, pig, cat, dog, horse, goat, cattle, and monkey and/or others. Most preferably, the patient is a human being.

An important embodiment of the present invention is the use of said lipid particles or liposomes containing amino lipids according to one of formulas (I-III) for use as a medicament.

In particular, said lipid particles or liposomes can be administered to patients for use in gene therapy, in gene vaccination, in antisense therapy or in therapy by interfering RNA. A lipid particle of the invention may also be used for the manufacture of a medicine for use in nucleic acid transfer, for example in treatment of the human or animal body by therapy, especially in the treatment of a condition caused by or related to a genetic defect or modification.

Targets for gene therapy are well known and include monogenic disorders, for example, cystic fibrosis, various cancers, and infections, for example, viral infections, for example, with HIV. For example, transfection with the p53 gene offers great potential for cancer treatment. Targets for gene vaccination are also well known, and include vaccination against pathogens for which vaccines derived from natural sources are too dangerous for human use and recombinant vaccines *are* not always effective, for example, hepatitis B virus, human immunodeficiency virus (HIV), hepatitis C virus (HCV) and herpes simplex virus.

Targets for anti-sense therapy are also known. Further targets for gene therapy and anti-sense therapy are being proposed as knowledge of the genetic basis of disease increases, as are further targets for gene vaccination.

A lipid particle of the invention may be used in vaccination. Thus, a lipid particle or liposome of the invention may be used to deliver an antigen or a nucleic acid encoding an antigen. These techniques are familiar to a person skilled in the art. Examples for liposome vaccines are described in Butts C, et al. Randomized phase IIB trial of BLP25 liposome vaccine in stage IIIB and IV Non-Small-Cell Lung Cancer. Journal of Clinical Oncology. 2005, 23:6674-6681 and in U'Ren L, et al. Vaccination with liposome-DNA complexes elicits enhanced antitumor immunity. Cancer Gene Therapy. 2006, 13:1033-1044.

A lipid particle of the invention may be used to elicit an immune response against a wide variety of antigens for the treatment and/or prevention of a number of conditions including, but not limited to, cancer, allergies, toxicity and infection by pathogens such as viruses, bacteria, fungi, and other pathogenic organisms.

In a further embodiment of the invention, said lipid particle or liposome can be used as a medicament in treatment of a viral infection, a liver disease or disorder, or cancer. On liver diseases, liposomes can be captured by the cells of the reticuloendothelial system, which are primarily situated in the liver. The liposome will be accumulated there.

### Figures

The following figures are presented to provide a better understanding of the description of procedures and conceptual aspects of the invention.
**Fig. 1****:** Comparative microscope pictures showing results of lipofection by use of amino liposomal reagent # 30 and one commercially available transfection reagent.
**Fig. 2****:** Comparative microscope pictures showing results of lipofection by use of an amino liposomal reagent #60 and one commercially available transfection reagent.
**Fig. 3****:** Graphical overview of the transfection efficiency of a library of transfection reagents of example compounds 1 to 44 compared to a commercially available reagent.
**Fig. 4****:** Graphical overview of the transfection efficiency of a library of transfection reagents of example compounds 47 to 76 compared to a commercially available reagent.
**Fig. 5****:** siRNA gene silencing in MEF cells using lipid reagent #24.
**Fig. 6****:** siRNA gene silencing in MEF cells using lipid reagent #64.

### Examples

The following 81 examples are presented to provide a better understanding of the description of procedures and conceptual aspects of the invention.

### Example 1: Synthesis and characterization of 2,3-bis(dodecylthio)propyl (2-(dimethylamino)ethyl) carbamate

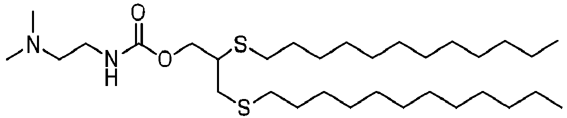

The amino lipid was synthesized by two steps. The first step is to synthesize prop-2-yn-1-yl (2-(dimethylamino)ethyl) carbamate. 0.1 mmol carbonyiimidazole and 0.1 mmol propargyl alcohol were dissolved in 2 ml DCM and added into a 20 ml glass vial covered with aluminium foil. The solution was shaked for 1 h, then 0.1 mmol dimethylethane-1,2-diamine and 0.01 mmol DMAP were added, and the solution was shaken overnight.

The second step is the photo addition of dodecanethiol on to prop-2-yn-1-yl (2-(dimethylamino)ethyl) carbamate. To do this, 0.2 mmol dodecanthiol and 3 mg DMPA were added to the solution from first step and the vial was degassed by argon and irradiated under 365 nm UV for 2h. The vial was then dried and stored under 4 °C. To verify the identity of the molecules, crude product was analyzed by mass spectrometry. The molecular ion was clearly identified as 574.46 W/z.

Synthesis of the compounds of the examples 2-22 were carried out similarly to example 1. Step 1 differs in the used amine and the stoichiometric ratios differ for examples 17-22. Step 2 differs in the educts, whereas the stoichiometric ratios were maintained. The resulting compounds and the corresponding MW/z values are summarized in Table 1:

**Table 1: Examples of synthesized compounds 2-22 and the corresponding MW/z values.**

| **Example** | **Compound** | **MW/z** |
|---|---|---|
| **2** | 2,3-bis(undecylthio)propyl (2-(dimethylamino)ethyl)carbamate | 546.43 |
| | 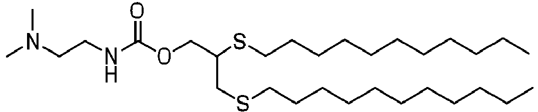 | |
| **3** | 2,3-bis(dodecylthio)propyl (2-(pyrrolidin-1-yl)ethyl)carbamate | 600.47 |
| | 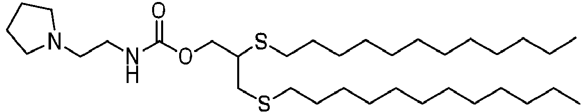 | |
| **4** | 2,3-bis(undecylthio)propyl (2-(pyrrolidin-1-yl)carbamate | 572.44 |
| | 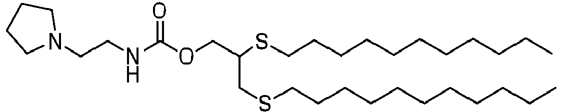 | |
| **5** | 2,3-bis(dodecylthio)propyl (2-(diethylamino)ethyl)carbamate | 602.49 |
| | 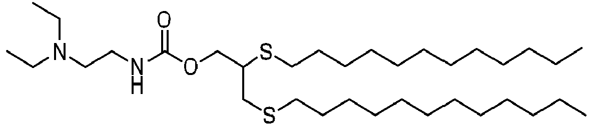 | |
| **6** | 2,3-bis(undecylthio)propyl (2-(diethylamino)ethyl)carbamate | 574.46 |
| | 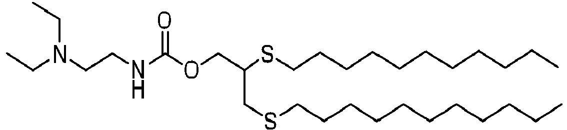 | |
| **7** | 2,3-bis(dodecylthio)propyl (3-(diethylamino)propyl)carbamate | 616.5 |
| | 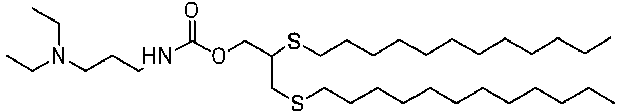 | |
| **8** | 2,3-bis(undecylthio)propyl (3-(diethylamino)propyl)carbamate | 588.47 |
| | 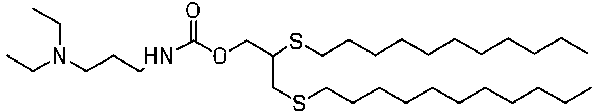 | |
| **9** | 2,3-bis(dodecylthio)propyl (3-(dimethylamino)propyl)carbamate | 588.47 |
| | 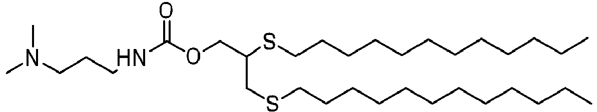 | |
| **10** | 2,3-bis(undecylthio)propyl (3-(dimethylamino)propyl)carbamate | 560.44 |
| | 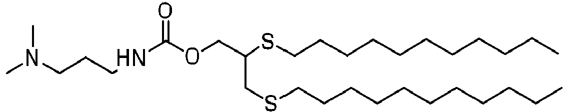 | |
| **11** | 3-(dodecylthio)propyl (2-(dimethylamino)ethyl)carbamate | 374.3 |
| | 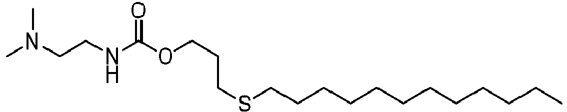 | |
| **12** | 3-(undecylthio)propyl (2-(dimethylamino)ethyl)carbamate | 360.28 |
| | 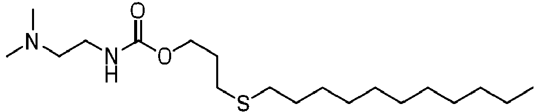 | |
| **13** | 3-(dodecylthio)propyl (2-(diethylamino)ethyl)carbamate | 402.33 |
| | 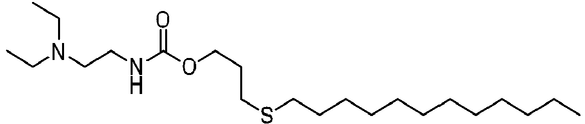 | |
| **14** | 3-(undecylthio)propyl (2-(diethylamino)ethyl)carbamate | 388.31 |
| | 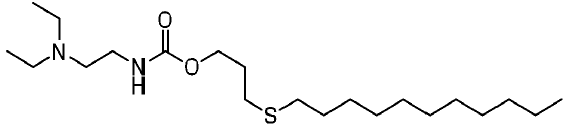 | |
| **15** | 3-(dodecylthio)propyl (3-(diethylamino)propyl)carbamate | 416.34 |
| | 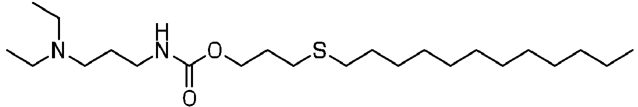 | |
| **16** | 3-(undecylthio)propyl (3-(diethylamino)propyl)carbamate | 402.33 |
| | 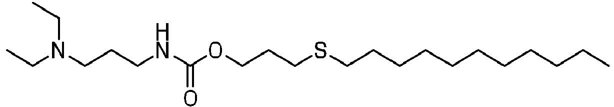 | |
| **17** | bis(3-(dodecylthio)propyl) (piperazine-1,4-diylbis(propane-3,1-diyl))dicarbamate | 772.59 |
| | 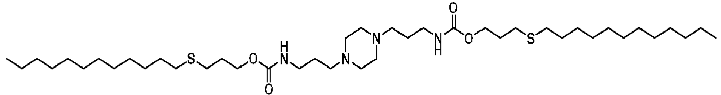 | |
| **18** | bis(3-(undecylthio)propyl) (piperazine-1,4-diylbis(propane-3,1-diyl))dicarbamate | 744.56 |
| | 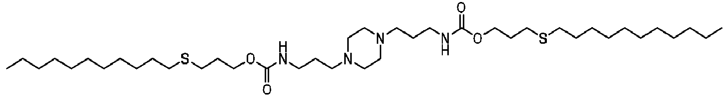 | |
| **19** | bis(3-(dodecylthio)propyl) ((methylazanediyl)bis(propane-3,1-diyl))dicarbamate | 717.55 |
| | 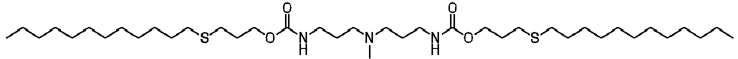 | |
| **20** | bis(3-(undecylthio)propyl) ((methylazanediyl)bis(propane-3,1-diyl))dicarbamate | 689.52 |
| | 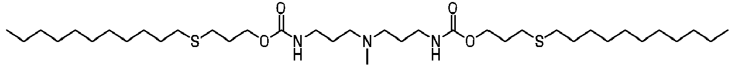 | |
| **21** | 3-(dodecylthio)propyl 4-(2-(((3-(dodecylthio)propoxy)carbonyl)amino)ethyl)piperazine-1-carboxylate | 701.52 |
| | 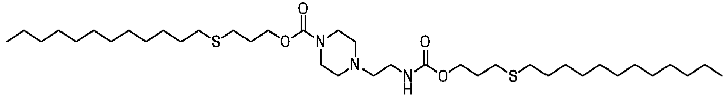 | |
| **22** | 3-(undecythio)propy 4-(2-(((3-(undecylthio)propoxy)carbonyl)amino)ethyl)piperazine-1-carboxylate | 673.49 |
| | 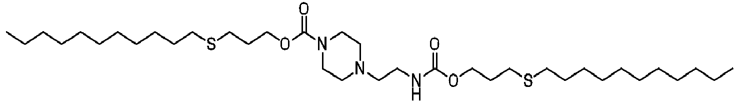 | |

### Example 23: Synthesis and characterization of 1-(2,3-bis(dodecylthio)propyl)-3-(2-(dimethylamino)ethyl)urea

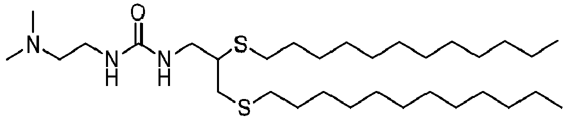

The synthesis procedure of 1-(2,3-bis(dodecylthio)propyl)-3-(2-imethylamino)ethyl)-urea is similar to the previous examples.

The first step is to synthesize 1-(2-(dimethylamino)ethyl)-3-(prop-2-yn-1-yl)urea. 0.1 mmol carbonyiimidazole and 0.1 mmol propargyl amine were dissolved in 2 ml DCM and added into a 20 ml glass vial covered with aluminium foil. The solution was shaked for 1 h, then 0.1 mmol dimethylethane-1,2-diamine and 0.01 mmol DMAP were added, the solution was shaked for overnight.

The second step is the photo addition of thiol onto 1-(2-(dimethylamino)ethyl)-3-(prop-2-yn-1-yl)urea. To do this, 0.2 mmol dodecanthiol and 3 mg DMPA were added to the solution from first step and the vial was degassed by argon and irradiated under 365 nm UV for 2h. The vial was then dried and stored under 4 °C.

To verify the identity of the molecules, crude product was analyzed by mass spectrometry. The molecular ion was clearly identified as 573.47 MW/z.

Syntheses according to the examples 24-44 were carried out similarly to example 23. Step 1 differs in the used amine as well as the stoichiometric ratio for the examples 39 - 44. Step 2 differs in the educts, whereas the stoichiometric ratios were maintained. The resulting compounds and the corresponding molecular ion are resumed in table 2:

**Table 2: Examples of synthesized compounds 24-44 and the corresponding MW/z values.**

| **Example** | **Compound** | **MW/z** |
|---|---|---|
| **24** | 1-(2,3-bis(undecylthio)propyl)-3-(2-(dimethylamino)ethyl)urea | 545.44 |
| | 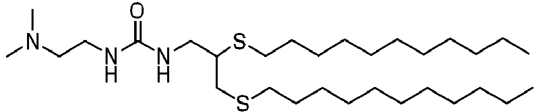 | |
| **25** | 1-(2,3-bis(dodecylthio)propyl)-3-(2-(pyrrolidin-1-yl)ethyl)urea | 599.49 |
| | 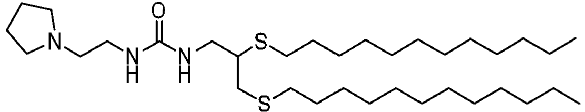 | |
| **26** | 1-(2,3-bis(undecylthio)propyl)-3-(2-(pyrrolidin-1-yl)ethyl)urea | 571.46 |
| | 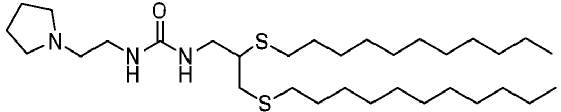 | |
| **27** | 1-(2,3-bis(dodecylthio)propyl)-3-(2-(diethylamino)ethyl)urea | 601.50 |
| | 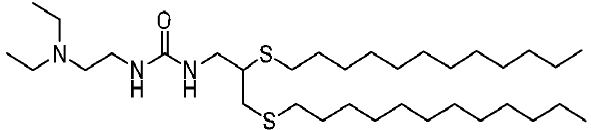 | |
| **28** | 1-(2,3-bis(undecylthio)propyl)-3-(2-(diethylamino)ethyl)urea | 573.47 |
| | 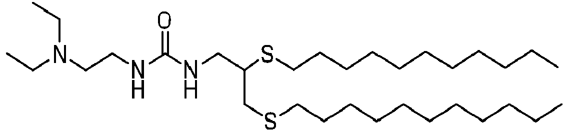 | |
| **29** | 1-(2,3-bis(dodecylthio)propyl)-3-(3-(diethylamino)propyl)urea | 615.52 |
| | 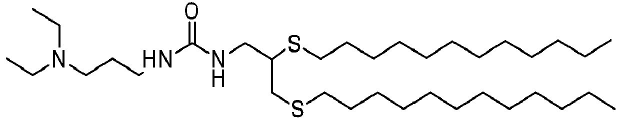 | |
| **30** | 1-(2,3-bis(undecylthio)propyl)-3-(3-(diethylamino)propyl)urea | 587.49 |
| | 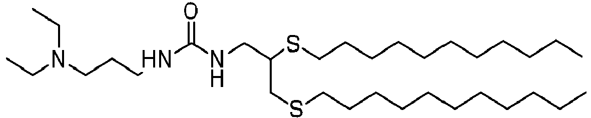 | |
| **31** | 1-(2,3-bis(dodecylthio)propyl)-3-(3-(dimethylamino)propyl)urea | 587.49 |
| | 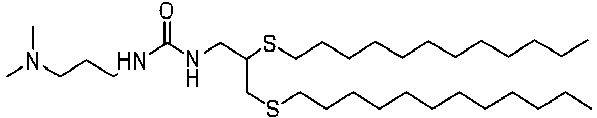 | |
| **32** | 1-(2,3-bis(undecylthio)propyl)-3-(3-(dimethylamino)propyl)urea | 559.46 |
| | 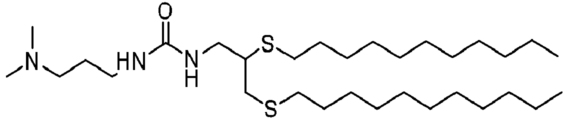 | |
| **33** | 1-(2-(dimethylamino)ethyl)-3-(3-(dodecylthio)propyl)urea | 373.31 |
| | 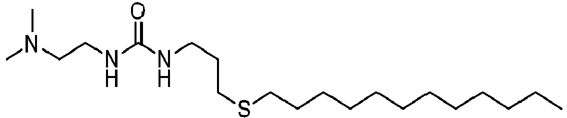 | |
| **34** | 1-(2-(dimethylamino)ethyl)-3-(2-(undecylthio)propyl)urea | 359.30 |
| | 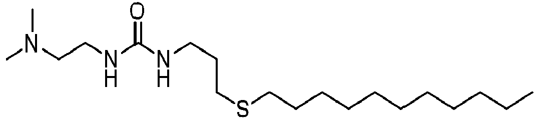 | |
| **35** | 1-(2-(diethylamino)ethyl)-3-(2-(dodecylthio)ethyl)urea | 401.34 |
| | 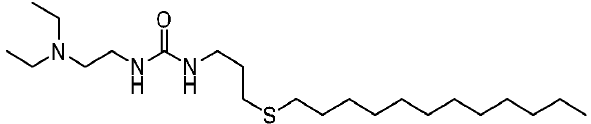 | |
| **36** | 1-(2-(diethylamino)ethyl)-3-(2-(undecylthio)propyl)urea | 387.33 |
| | 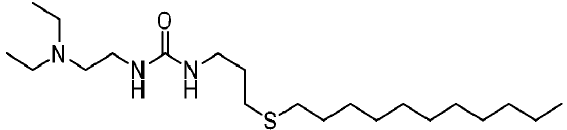 | |
| **37** | 1-(3-(diethylamino)propyl)-3-(3-(dodecylthio)propyl)urea | 415.36 |
| | 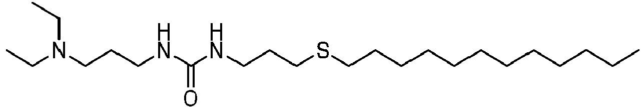 | |
| **38** | 1-(3-(diethylamino)propyl)-3-(3-(undecylthio)propyl)urea | 401.34 |
| | 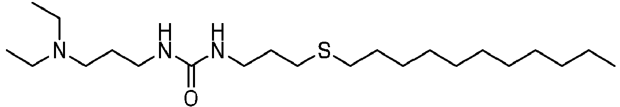 | |
| **39** | 1,1'-(piperazine-1,4-diylbis(propane-3,1-diyl))bis(3-(3-(dodecylthio)propyl)urea) | 770.63 |
| | 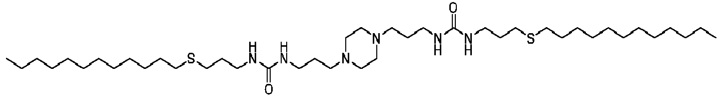 | |
| **40** | 1,1'-(piperazine-1,4-diylbis(propane-3,1-diyl))bis(3-(3-(undecylthio)propyl)urea) | 742.59 |
| | 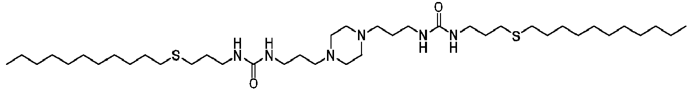 | |
| **41** | 1,1'-((methylazanediyl)bis(propane-3,1-diyl))bis(3-(3-(dodecylthio)propyl)urea) | 715.58 |
| | 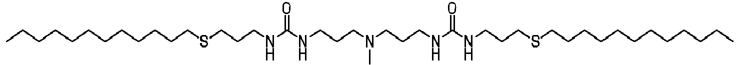 | |
| **42** | 1,1'-((methylazanediyl)bis(propane-3,1-diyl))bis(3-(3-(undecylthio)propyl)urea) | 687.55 |
| | 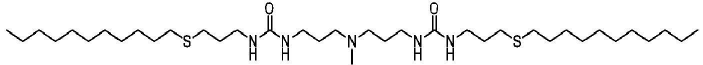 | |
| **43** | N-(3-(dodecylthio)propyl)-4-(2-(3-(3-(dodecylthio)propyl)ureido)ethyl)piperazine-1-carboxamide | 699.55 |
| | 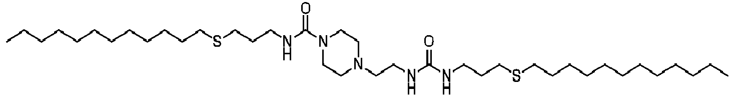 | |
| **44** | N-(3-(undecylthio)propyl)-4-(2-(3-(3-(undecylthio)propyl)ureido)ethyl)piperazine-1-carboxamide | 671.52 |
| | 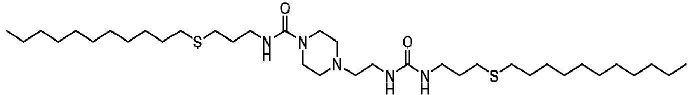 | |

### Example 45: Synthesis and characterization of 3-(dodecylsulfinyl)-2-(dodecylthio)propyl (2-(dimethylamino)ethyl)carbamate

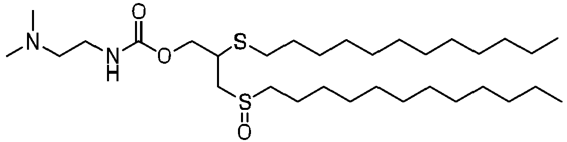

The amino lipid is synthesized in one step. 1 mmol 2,3-bis(dodecylthio)propyl (2-(dimethylamino)ethyl)carbamate (product of example 1) was mixed with 10 mmol aqueous hydrogen peroxide (30%) in 10 ml methanol, and stirred at room temperature for 1 h. Then the mixture was transferred to a 50 ml flask and evaporated.

To verify the identity of the molecules, crude product was analyzed by mass spectrometry. The molecular ion was clearly identified as 590.5 MW/z.

### Example 46: Synthesis and characterization of 2,3-bis(dodecylsulfonyl)propyl (2-(dimethylamino)ethyl)carbamate

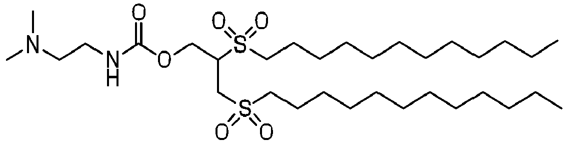

The amino lipid is synthesized in one step. 1 mmol 2,3-bis(dodecylthio)propyl (2-(dimethylamino)ethyl)carbamate (product of example 1) was mixed with 10 mmol aqueous hydrogen peroxide (30%) in 10 ml methanol, and stirred at room temperature for 2 d. Then the mixture was transferred to a 50 ml flask and evaporated.

To verify the identity of the molecules, crude product was analyzed by mass spectrometry. The molecular ion was clearly identified as 638.4 MW/z.

### Example 47: Synthesis and characterization of N¹,N¹-bis(3-(dodecylthio)propyl)-N²,N²-dimethylethane-1,2-diamine

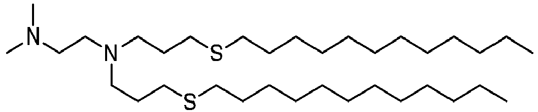

The synthesis procedure of N¹,N¹-bis(3-(dodecylthio)propyl)-N²,N²-dimethylethane-1,2-diamine is similar to the previous examples. 1 mmol of N¹,N¹-dimethylethane-1,2-diamine, and 2 mmol 3-bromoprop-1-ene were added to a 20 ml glass vial containing 2 ml THF and 1 wt % Cs₂CO₃. The solution was shaked for overnight at 40°C. The solution vial was then removed from the shaker, centrifuged and the supernatant was transferred into a new 20 ml vial. 2 mmol dodecane-1-thiol and 5-7 wt% 2,2-Dimethoxy-2-phenylacetonphenone (DMPA) solution in THF were added to the above 20 ml glass vial. The mixture was irradiated under UV 365 nm for 2 h and then the THF was evaporated out.

This step was carried out similar to the previous examples. The stoichiometric ratios were maintained.

To verify the identity of the molecules, crude product was tested by mass spectrometry (m/z 572.51).

Syntheses according to the examples 48-59 were carried out similarly to example 47. Step 1 differs in the used amine, whereas the stoichiometric ratios were maintained. The resulting compounds and the corresponding molecular ion are resumed in table 3.

**Table 3: Examples of synthesized compounds 48-56 and the corresponding MW/z values.**

| **Example** | **Compound** | **MW/z** |
|---|---|---|
| **48** | 3-(dodecylthio)-N-(3-(dodecylthio)propyl)-N-(2-(pyrrolidin-1-yl)ethyl)propan-1-amine | 598.52 |
| | 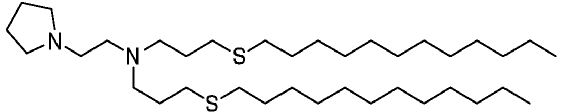 | |
| **49** | N¹,N¹-bis(3-(dodecylthio)propyl)-N²,N²-diethylethane-1,2-diamine | 600.54 |
| | 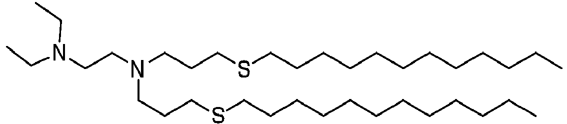 | |
| **50** | N¹,N¹-bis(3-(dodecylthio)propyl)-N³,N³-diethylpropane-1,3-diamine | 614.56 |
| | 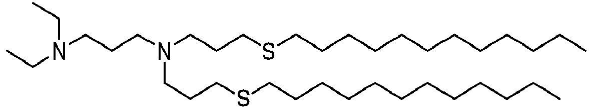 | |
| **51** | N¹,N¹-bis(3-(dodecylthio)propyl)-N³,N³-dimethylpropane-1,3-diamine | 586.52 |
| | 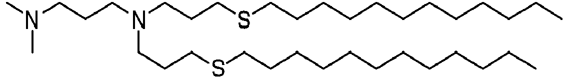 | |
| **52** | N¹,N¹-dimethyl-N²,N²-bis(3-(undecylthio)propyl)ethane-1,2-diamine | 544.48 |
| | 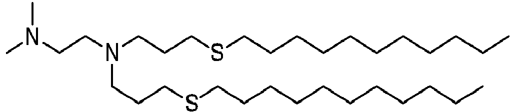 | |
| **53** | N-(2-(pyrrolidin-1-yl)ethyl)-3-(undecylthio)-N-(3-(undecylthio)propyl)propan-1-amine | 570.50 |
| | 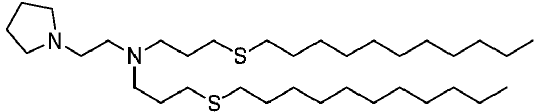 | |
| **54** | N¹,N¹-diethyl-N²,N²-bis(3-(undecylthio)propyl)ethane-1,2-diamine | 572.51 |
| | 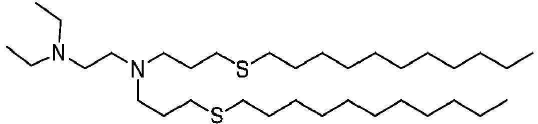 | |
| **55** | N¹,N¹-diethyl-N³,N³-bis(3-(undecylthio)propyl)propane-1,3-diamine | 586.53 |
| | 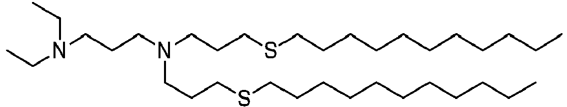 | |
| **56** | N¹,N¹-dimethyl-N³,N³-bis(3-(undecylthio)propyl)propane-1,3-diamine | 558.50 |
| | 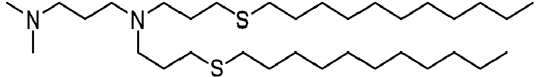 | |

### Example 57: Synthesis and characterization of 11-(dodecylthio)-2-methyl-9-oxa-13-thia-2,5-diazapentacosan-7-ol

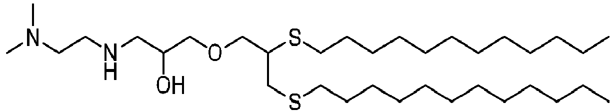

The lipid was synthesized as follows, 1 mmol N¹,N¹-dimethylethane-1,2-diamine, and 1 mmol 2-((prop-2-yn-1-yloxy)methyl)oxirane were dissolved in 2 ml ethanol in a 20 ml glass vial covered with septum. The solution was shaked for overnight at 40°C. The solution vial was then removed from the shaker, ethanol was evaporated and 2 ml fresh THF was added and mixed well, the solution was then degassed for 5 min and filled with Argon (Ar) for 3 min, then covered with a cap. 2 mmol dodecane-1-thiol and 5-7 wt% 2,2-Dimethoxy-2-phenylacetonphenone (DMPA) solution in THF were added to the above 20 ml glass vial. The mixture was irradiated under UV 365 nm for 2 h and then the THF was evaporated out.

To verify the identity of the molecules, crude product was analyzed by mass spectrometry. The molecular ion was clearly identified as 605.08 MW/z.

Synthesis of the compounds of the examples 58-66 were carried out similarly to example 57. The resulting compounds and the corresponding MW/z values are summarized in Table 4:

**Table 4: Examples of synthesized compounds 58-66 and the corresponding MW/z values.**

| **Example** | **Compound** | **MW/z** |
|---|---|---|
| **58** | 1-(2,3-bis(dodecylthio)propoxy)-3-((2-(pyrrolidin-1-yl)ethyl)amino)propan-2-ol | 631.11 |
| | 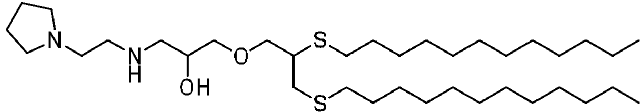 | |
| **59** | 12-(dodecylthio)-3-ethyl-10-oxa-14-thia-3,6-diazahexacosan-8-ol | 633.13 |
| | 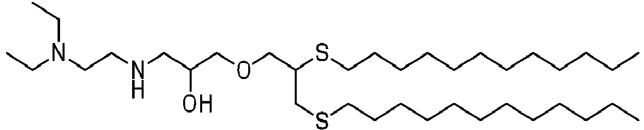 | |
| **60** | 13-(dodecylthio)-3-ethyl-11-oxa-15-thia-3,7-diazaheptacosan-9-ol | 647.16 |
| | 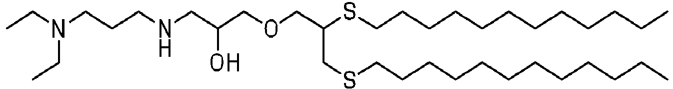 | |
| **61** | 12-(dodecylthio)-2-methyl-10-oxa-14-thia-2,6-diazahexacosan-8-ol | 619.10 |
| | 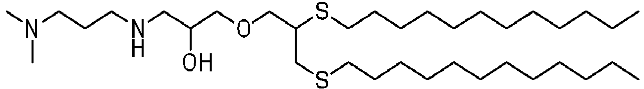 | |
| **62** | 2-methyl-11-(undecylthio)-9-oxa-13-thia-2,5-diazatetracosan-7-ol | 577.02 |
| | 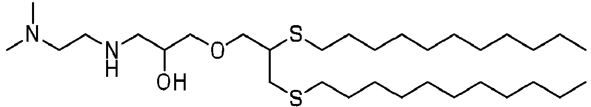 | |
| **63** | 1-(2,3-bis(undecylthio)propoxy)-3-((2-(pyrrolidin-1-yl)ethyl)amino)propan-2-ol | 603.06 |
| | 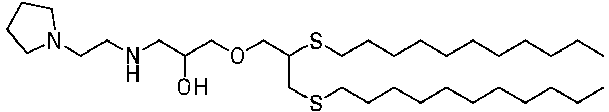 | |
| **64** | 3-ethyl-12-(undecylthio)-10-oxa-14-thia-3,6-diazapentacosan-8-ol | 605.08 |
| | 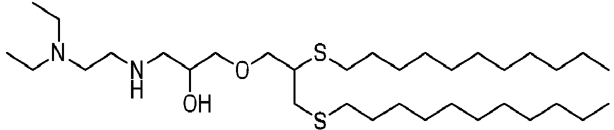 | |
| **65** | 3-ethyl-13-(undecylthio)-11-oxa-15-thia-3,7-diazahexacosan-9-ol | 619.10 |
| | 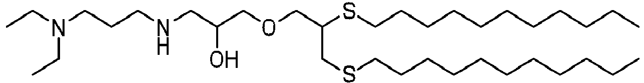 | |
| **66** | 2-methyl-12-(undecylthio)-10-oxa-14-thia-2,6-diazapentacosan-8-ol | 519.05 |
| | 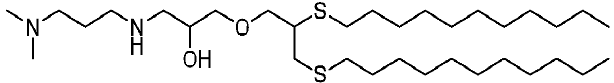 | |

### Example 67: Synthesis and characterization of 21-(2-(dimethylamino)ethyl)-17,25-dioxa-13,29-dithia-21-azahentetracontane-19,23-diol

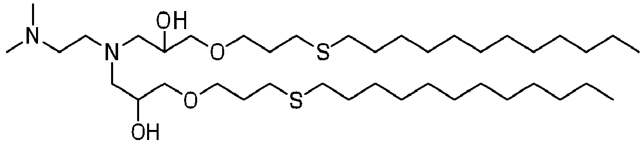

The synthesis procedure of 21-(2-(dimethylamino)ethyl)-17,25-dioxa-13,29-dithia-21-azahentetracontane-19,23-diol is similar to the previous examples. 1 mmol N¹,N¹-dimethylethane-1,2-diamine, and 2 mmol 2-((allyloxy)methyl)oxirane were dissolved in 2 ml ethanol in a 20 ml glass vial covered with septum. The solution was shaken for overnight at 40°C. The solution vial was then removed from the shaker, ethanol was evaporated and 2 ml fresh THF was added and mixed well, the solution was then degassed for 5 min and filled with Argon (Ar) for 3 min, then covered with a cap. 2 mmol dodecane-1-thiol and 5-7 wt% 2,2-Dimethoxy-2-phenylacetonphenone (DMPA) solution in THF were added to the above 20 ml glass vial. The mixture was irradiated under UV 365 nm for 2 h and then the THF was evaporated out.

This step was carried out similar to the previous examples. The stoichiometric ratios were maintained.

To verify the identity of the molecules, crude product was tested by mass spectrometry (m/z 721.24).

Synthesises according to the examples 68-76 were carried out similarly to example 67. The resulting compounds and the corresponding molecular ion are resumed in table 5:

**Table 5: Examples of synthesized compounds 68-76 and the corresponding MW/z values.**

| **Example** | **Compound** | **MW/z** |
|---|---|---|
| **68** | 21-(2-(pyrrolidin-1-yl)ethyl)-17,25-dioxa-13,29-dithia-21-azahentetracontane-19,23-diol | 747.27 |
| | 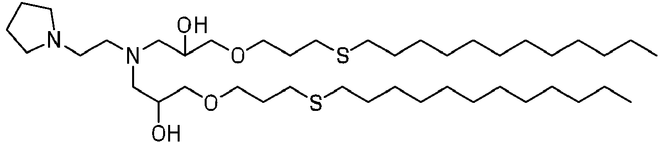 | |
| **69** | 21-(2-(diethylamino)ethyl)-17,25-dioxa-13,29-dithia-21-azahentetracontane-19,23-diol | 749.29 |
| | 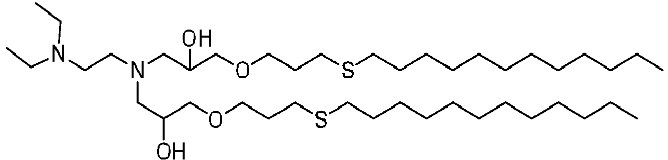 | |
| **70** | 21-(4-(diethylamino)butyl)-17,25-dioxa-13,29-dithia-21-azahentetracontane-19,23-diol | 777.34 |
| | 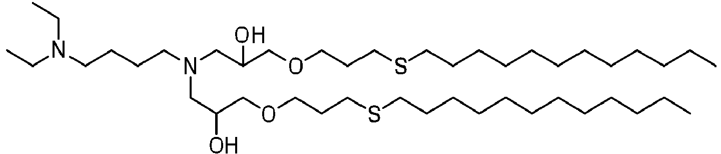 | |
| **71** | 21-(4-(dimethylamino)butyl)-17,25-dioxa-13,29-dithia-21-azahentetracontane-19,23-diol | 749.29 |
| | 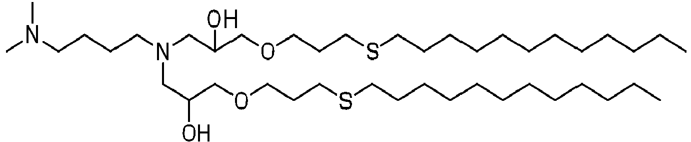 | |
| **72** | 20-(2-(dimethylamino)ethyl)-16,24-dioxa-12,28-dithia-20-azanonatriacontane-18,22-diol | 693.18 |
| | 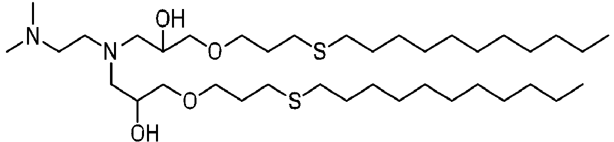 | |
| **73** | 20-(2-(pyrrolidin-1-yl)ethyl)-16,24-dioxa-12,28-dithia-20-azanonatriacontane-18,22-diol | 719.12 |
| | 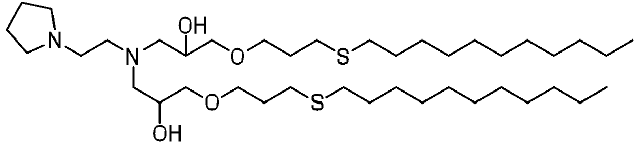 | |
| **74** | 20-(2-(diethylamino)ethyl)-16,24-dioxa-12,28-dithia-20-azanonatriacontane-18,22-diol | 721.24 |
| | 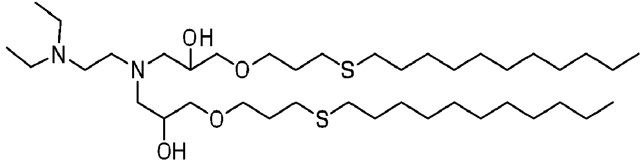 | |
| **75** | 20-(4-(diethylamino)butyl)-16,24-dioxa-12,28-dithia-20-azanonatriacontane-18,22-diol | 749.29 |
| | 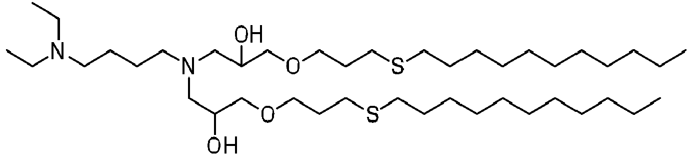 | |
| **76** | 20-(4-(dimethylamino)butyl)-16,24-dioxa-12,28-dithia-20-azanonatriacontane-18,22-diol | 721.24 |
| | 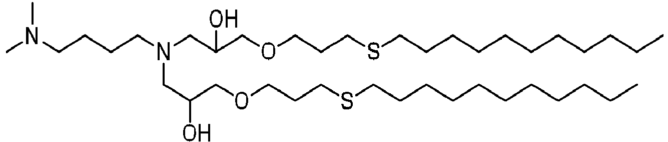 | |

### Example 77: Synthesis and characterization of N¹-(3-(dodecylsulfinyl)-2-(dodecylthio)propyl)-N²,N²-dimethylethane-1,2-diamine

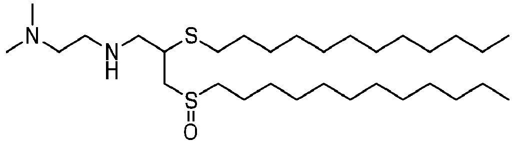

The amino lipid is synthesized in one step. 1 mmol N¹-(2,3-bis(dodecylthio)propyl)-N²,N²-dimethylethane-1,2-diamine (product of example 50) was mixed with 10 mmol aqueous hydrogen peroxide (30%) in 10 ml methanol, and stirred at room temperature for 1 h. Then the mixture was transferred to a 50 ml flask and evaporated.

To verify the identity of the molecules, crude product was analyzed by mass spectrometry. The molecular ion was clearly identified as 546.5 MW/z.

### Example 78: Synthesis and characterization of N¹-(2,3-bis(dodecylsulfonyl)propyl)-N²,N²-dimethylethane-1,2-diamine

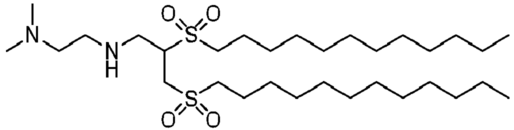

The amino lipid is synthesized in one step. 1 mmol N¹-(2,3-bis(dodecylthio)propyl)-N²,N²-dimethylethane-1,2-diamine (product of example 50) was mixed with 10 mmol aqueous hydrogen peroxide (30%) in 10 ml methanol, and stirred at room temperature for 2 d. Then the mixture was transferred to a 50 ml flask and evaporated.

To verify the identity of the molecules, crude product was analyzed by mass spectrometry. The molecular ion was clearly identified as 594.4 MW/z.

### Screening of the cationic lipid for cell transfection

### Example 79: Initial determination of optimal lipid ratios for cell transfection.

The well documented HEK 293T cell line is used for examples 79 and 80.

The natural phospholipid dioleolylphosphatidylethanolamine (DOPE - structure shown below) was selected as the required co-lipid (also termed helper lipid). It is required not for the stability of liposomes per se, but rather the breakdown of the lipid membranes in the endocytic compartment (endosomes) of cells, allowing release of the bioactive agent to the cytosol and/or nucleus. Basically, it is required for the desired effect for stable liposome formation in combination with our cationic amino lipids (DPDEC). DOPE was mixed with a representative cationic amino lipid (structure shown below) in different ratios. Both lipids were dissolved in ethanol at 50 mg/ml and combined to a final volume of 30 µl.

DOPE (co-lipid):

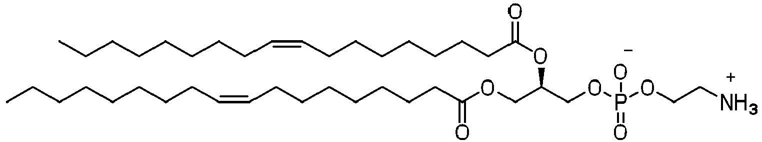

2,3-bis(dodecylthio)propyl (2-(dimethylamino)ethyl) carbamate (DPDEC) as representative novel cationic amino lipids:

### DPDEC:

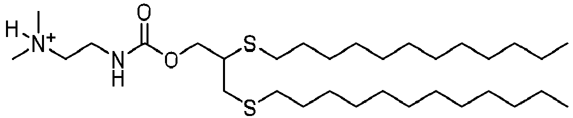

**Table 6: DPDEC ratios tested**

| **DPDEC** | **DOPE** |
|---|---|
| 0 | 1 |
| 1 | 3 |
| 1 | 2 |
| 1 | 1 |
| 2 | 1 |
| 3 | 1 |
| 1 | 0 |

These 30 µl ethanol mixtures were then added to 70 µl of 0,2 M Sodium acetate buffer (pH 5.0) with constant vortexing for 30 s, followed by sonication for 5 min to form liposomes. Final lipid content is 2 mg/ml. This final 2 mg/ml liposome sample is referred to as the "lipid reagent".

0.1 µl, 0.2 µl, 0.3 µl, 0.4 µl and 0.5 µl of the above lipid reagents were combined with either 50 ng or 100 ng plasmid DNA (comprising the pCS-LacZ and pH2B-YFP plasmids at a ratio of 5:1, respectively) and mixed with cells as described below:
(amounts shown are for one well of a 96-well culture plate)
**1.** 0.1µl-0.5 µl lipid reagent diluted in 10µl of 50 mmol/l sodium acetate buffer, pH 5.0.
**2.** After 2-5 min incubation, added diluted lipid reagent from (1) to either 50ng or 100ng plasmid DNA (DNA dissolved in 10ul of 50 mmol/l sodium acetate buffer, pH 5.0).
**3.** Samples were left at RT for 30 min to form Lipid/DNA transfection complexes. As DNA is negatively charged, it associates non-specifically with the positively charged head groups of the cationic lipids in the liposomes.
**4.** After 30 min, 50 µl of freshly suspended HEK 293 cells (approximately 50,000 cells, in DMEM culture medium supplemented with 10% fetal calf serum) were added to the lipid/DNA complexes, mixed with pipette action and 65 µl of the cells + lipid/DNA complexes added to a single 96-well.

To assess the ability of the lipid mixtures to deliver the plasmid DNA into cells, microscopy was used to visualize fluorescence emitted by the yellow fluorescent protein (YFP) 20-24 hours after initial transfection. The YFP protein is encoded by the pH2B-YFP plasmid and is efficiently synthesized and located within the nucleus of successfully transfected cells.

### RESULTS:

A very clear optimal ratio of lipid: DOPE was identified as 2 : 1 and the optimal lipid reagent : DNA ratio was 0.4 µl lipid reagent per 75 ng DNA. These conditions were therefore used for the primary screen to identify the lipid reagents with highest cell transfection efficiency and lowest cell toxicity, as described in example 80 below.

### Example 80: Primary screen using novel lipids reagents

Cell line: HEK 293 cells

Screen format: 96-well

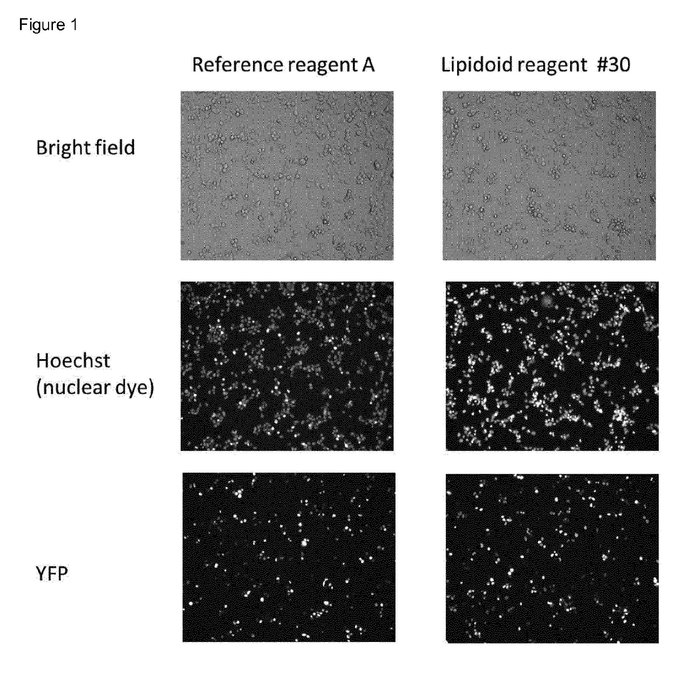

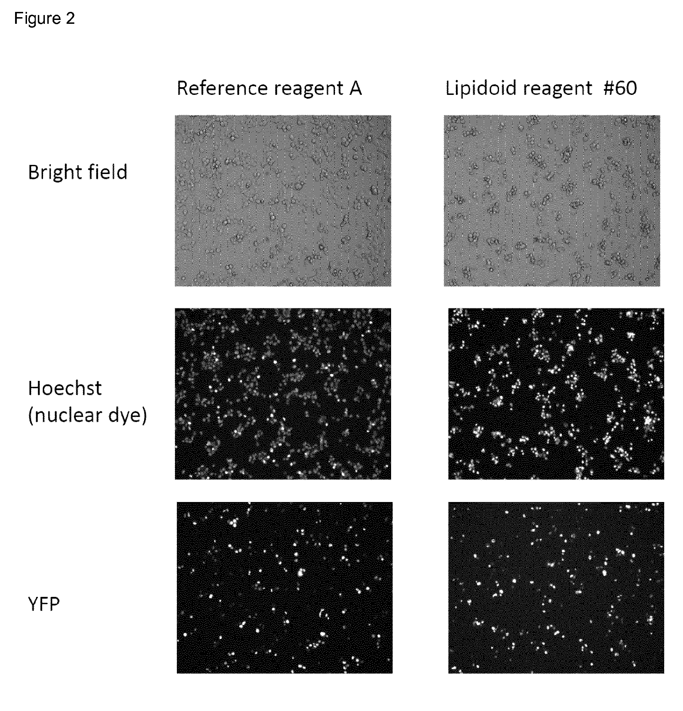

Detection (read-out): YFP fluorescence relative to total cell number (total cell number assessed using the nuclear dye, Hoechst - see Fig. 1 and 2)

A commercially available liposomal transfection reagent was used as a reference (reference reagent) according to manufacturers' instructions, see Fig. 1 and 2.

### Method:

All steps performed in 96 tube/plate format using 8- or 12-channel multi-pipettes. Amounts shown are for duplicate (2x) wells of a 96-well plate.
1. 0.8 µl lipid reagents diluted in 20 µl 50 mM NaOAc buffer, pH 5.0.
2. Diluted lipid reagents from (1) were added to 150ng DNA (25 ng pH2B-YFP + 125 ng pCS-LacZ plasmids) in 20 µl NaOAc buffer, pH 5.0 and mixed with pipette action.
3. After 30 min incubation at RT, added 100µl freshly resuspended cells (5 x 10⁴ cells/well DMEM culture medium supplemented with 10 % fetal calf serum) and mixed with pipette.
4. Duplicate 65µl aliquots of the cells + lipid/DNA complexes were immediately transferred to separate wells of a 96-well culture plate and placed in 37°C incubator with 5 % CO₂.
5. 20 to 24 hours after initial transfection of cells, Hoechst 33258 dye was added to cells at a final concentration of 0.2 µg/ml and cells incubated for a further 30 min at 37°C. Cells were then placed on an inverted microscope and 2 independent sets of images of the cells captured from each well as shown in Fig. 1 and 2.

For each sample, 3 images were captured: bright field image of cells (Fig. 1 and 2 upper panels), Hoechst dye stained image of total cell nuclei (Fig. 1 and 2 middle panels) and YFP images showing cells successfully transfected with plasmid DNA and expressing YFP protein (Fig. 1 and 2 lower panels). Microscope images of transfected HEK 293 cells, showing both the transfection efficiency and toxicity level of two of our lipid molecules (#30 and #60) compared to a commonly used commercial transfection reagent (reference reagent). Lipid reagents #30 and #60 have a transfection efficiency of approximately 90% and have low cell toxicity (very few brightly stained apoptotic nuclei).

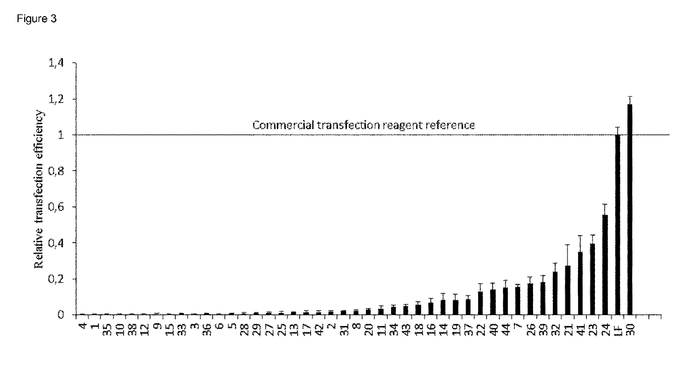

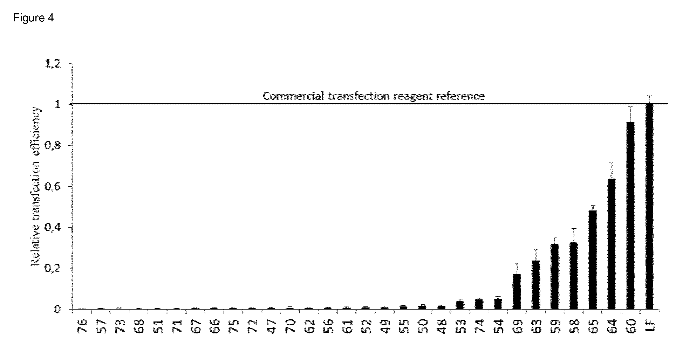

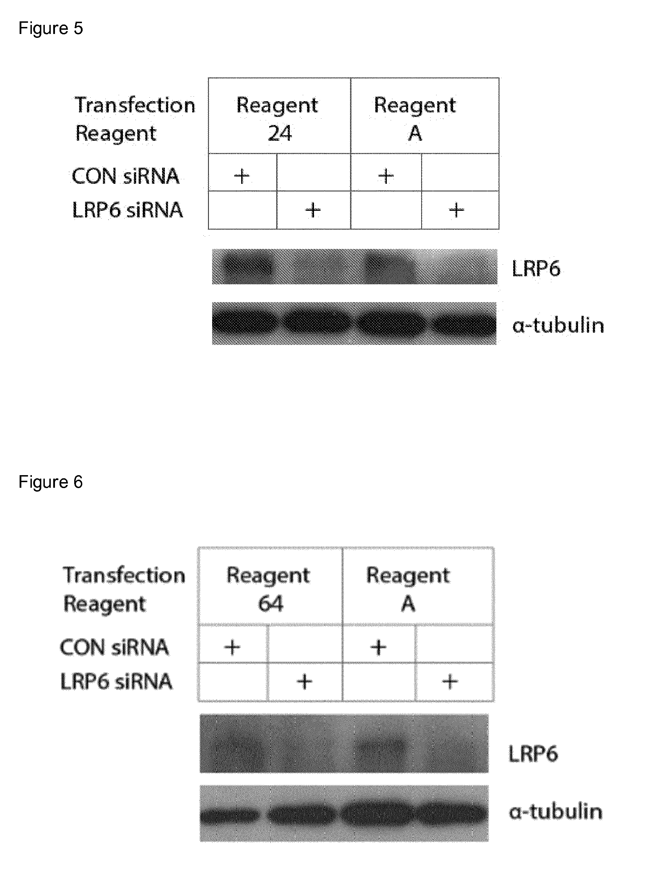

According to the protocol given in Example 80, a library of newly synthesized compounds according to claim 1 have been tested for their ability to transfect HEK 293 cells. The graphs in Fig. 3 and 4 show the transfection efficiency of these lipid compounds compared to a commercially available transfection agent reference. 1 of the lipid molecules is efficient at delivering plasmid DNA (YFP gene) to HEK 293 cells when compared to a widely used commercial transfection reagent, indicated by the solid line. Two reagents, in particular have been identified as possessing very low toxicity and have the ability to very efficiently deliver siRNA molecules to cells (#24, see Fig. 5 and #64; see Fig. 6).

### Example 81: Screen of library "hits" for ability to transfect siRNA

One of the key technologies for manipulation of gene function, both in cells and whole organisms, is gene silencing through RNA interference (RNAi). Delivery of small interfering RNA (siRNA) molecules into mammalian cells is crucial for this technology and has significant clinical/therapeutic implications.

Thus, in addition to screening our lipids for delivery of plasmid DNA (the genes) we have also screened amino lipids according to the present invention for their ability to efficiently deliver siRNA molecules (the gene silencers).

In order to screen for this two different types of cells were used to test the ability of our lipid reagents to deliver siRNA targeting Low density lipoprotein receptor related protein 6 (LRP6). This is a 200 kD single-pass transmembrane receptor for Wnt ligands and activates the Wnt/b-catenin signalling pathway. It is expressed at relatively high levels in MEF cells.

Assay: Transfection of siRNA in mouse embryonic fibroblast (MEF) cells

### Method:

All steps performed in 0.2ml PCR tubes and 96 well plate format. Amounts shown are for one well of a 96-well plate.
1. 0.25µl lipid reagents diluted in 8 µl 50 mmol/l NaOAc buffer, pH 5.0.
2. Diluted lipid reagents from (1) added to 1 pmol (0.2 µl of 5 µmol/l) siRNA molecules in 8 µl NaOAc buffer, pH 5.0 and mixed with pipette action. The siRNA molecules used had either a scrambled sequence not specific for any known gene (Con siRNA), or a sequence specifically targeting the endogenous mRNA from the *LRP6* gene *(LRP6* siRNA).
3. After 30 min incubation at RT, added 50µl freshly resuspended cells (2 x 10⁴ cells/well DMEM culture medium supplemented with 10% fetal calf serum) in one well of a 96-well plate and placed in 37 °C incubator with 5 % CO₂.
4. 24 hours after initial transfection, cells, were lysed in 25 µl detergent buffer (50 mmol/l Tris, 1% Triton X-100, 0,15 mol/l NaCl, pH 7.0, containing protease and phosphatase inhibitors), spun to remove insoluble cell debris and 30 µl clarified lysates added to 10 µl of 4x SDS loading buffer (250 mmol/l Tris HCl, 40 % Glycerol, 8 % SDS, 0.01 % Bromophenol Blue, 5 % 2-Mercaptoethanol, pH 6.8).
5. Samples were denatures by heating at 96 °C for 2 min and 12 µl loaded on a 9 % SDS-PAGE gels for separation of proteins according to molecular weight. Separated proteins were transferred from the SDS-PAGE gel to nitrocellulose membrane for Western Blot (WB) analysis.
6. WB was performed using an automated BioLane HTI instrument using antibodies against LRP6 and α-tubublin proteins. HRP linked secondary antibodies and chemiluminescence were used to detect the proteins on the membrane.

### Results

Figures 5 and 6 show the Western Blot (WB) analysis of LRP6 protein from total lysates of MEF (mouse embryonic fibroblast) cells transfected with the indicated siRNA molecules and cultured for 24 hours. Lipid reagents #24 and #64 are quite effective compared with the commercial reagent A at siRNA mediated gene silencing. This demonstrates the striking difference in properties of related transfection reagents and highlights the importance of using our novel method to easily synthesize hundreds of related lipids that can be screened to identify the ones having optimal properties (such as highly efficient DNA and siRNA delivery as well as low cellular toxicity).

### List of abbreviations

- Ar: Argon
- DCM: Dichloromethane
- DEDPA: N-(2-(Dimethylamino)ethyl)-4,5-bis(dodecylthio)pentanamide
- DIC: N, N'-Diisopropylcarbodiimide
- DMEM: Culture medium
- DMF: Dimethylformamide
- DMPA: 2,2-Dimethoxy-2-phenylacetonphenone
- DNA: Desoxyribonucleic acid
- DOPE: Dioleolylphosphatidylethanolamine
- YFP: Yellow Fluorescent Protein
- HOBt: Hydroxylbenzotriazole
- HRP: Horseradish Peroxidase
- kD: kilo Dalton
- LRP6: Low density lipoprotein receptor related protein 6
- MEF: Mouse embryonic fibroblast
- PEG: Polyethylene glycol
- RNA: Ribonucleic acid
- RNAi: RNA interference
- siRNA: small interfering RNA
- SDS: Sodium dodecyl sulfate
- THF: Tetrahydrofuran
- WB: Western Blot
- Wnt: Signalling proteins in cell differentiation

## Claims

1. Amino lipid comprising a structure of the following formula: 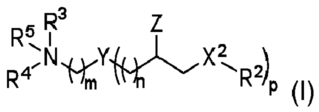 wherein Z is either hydrogen or -X¹R¹,
R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either 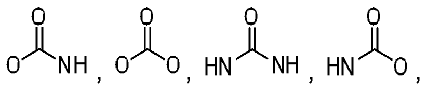 NH, NR⁷, ⁺NR⁶R⁷, 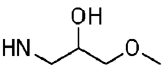 or heterocycles of the formula 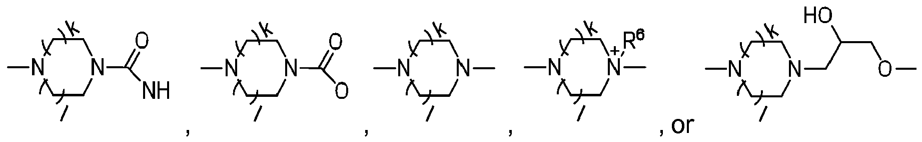 wherein k and l are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ optionally join to form an optionally substituted heterocyclic ring of 3 to 10 atoms and 0 to 6 heteroatoms selected from the group consisting of nitrogen, sulfur and oxygen, or R³ and R⁴ are the same or different alkyl amines;
R⁵ and R⁶ are either absent or is hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein preferably Y = N for p = 2 or 3.

2. The amino lipid of claim 1 having the structure of formula (Ia) or (Ib). 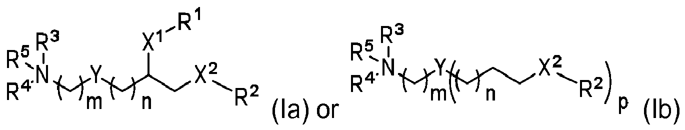 wherein R¹ and R² are the same or different and independently C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂,
Y is either 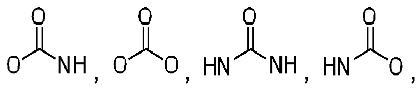 NH, NR⁷, ⁺NR⁶R⁷, 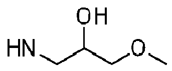 or heterocycles of the formula 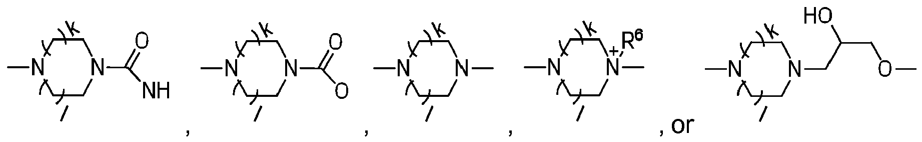 wherein k and l are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ optionally join to form an optionally substituted heterocyclic ring of 3 to 10 atoms and 0 to 6 heteroatoms selected from the group consisting of nitrogen, sulfur and oxygen, or R³ and R⁴ are the same or different alkyl amines;
R⁵ and R⁶ are either absent or is hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein preferably, Y = N for p = 2 or 3.

3. Amino lipids comprising a structure of the following formula (Ic) 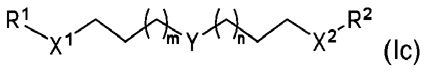 wherein R¹ and R² are the same or different and independently from each other C₆-C₂₄ alkyl, C₆-C₂₄ alkenyl, C₆-C₂₄ alkynyl, or C₆-C₂₄ acyl, which can be optionally substituted with a C₁-C₆ hydrocarbyl group,
X¹ and X² are the same or different, either S or S=O or S(=O)₂, Y is either 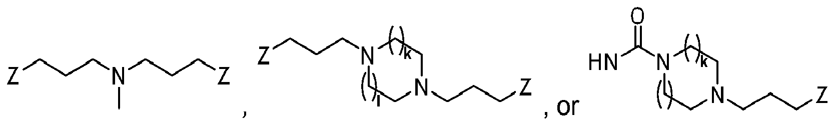 wherein Z is 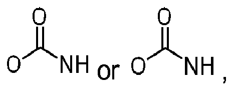 k and l are integers from 0 to 2.
m is an integer from 1 to 12 and n is an integer from 1 to 12.

4. The amino lipid of claim 1 or 2, wherein X¹ and X² are either the same or different and independently S=O or S(=O)₂.

5. The amino lipid of claim 1 to 4, wherein R¹ and R² are the same or different and independently C₆-C₂₄ alkyl, which is optionally substituted with a C₁-C₆ hydrocarbyl group.

6. The amino lipid of claim 1 or 5, having the structure of formula (IIa) or (IIb) 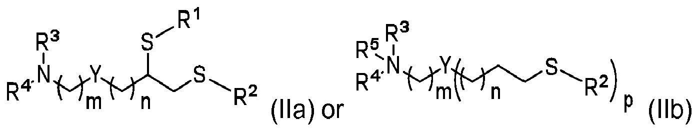 wherein R¹ and R² are the same C₆-C₁₈ alkyl,
Y is either 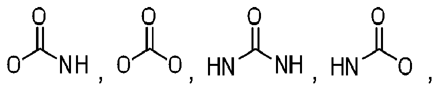 NH, NR⁷, ⁺NR⁶R⁷, 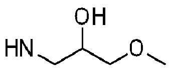 or heterocycles of the formula 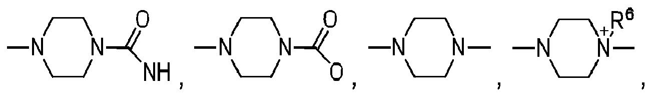 or 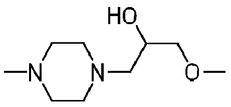 R³ and R⁴ are the same or different and independently C₁-C₁₂ alkyl, wherein alkyl is optionally substituted with a C₁-C₆ hydrocarbyl group, or R³ and R⁴ optionally join to form an optionally substituted heterocyclic ring of 3 to 10 atoms and 0 to 6 heteroatoms selected from the group consisting of nitrogen, sulfur and oxygen, or R³ and R⁴ are the same or different alkyl amines;
R⁵ and R⁶ are either absent or hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein preferably, Y = N for p = 2 or 3.

7. The amino lipid of claim 1, 5 or 6, having the structure of formula (IIIa), (IIIb), (IIIc), (IIId), (IIIe), (IIIf), (IIIg), (IIIh), (IIIi), (IIIj), (IIIk), (IIIl), (IIIm), (IIIn), (IIIo), (IIIp), (IIIq), (IIIr), (Ills), (IIIt), (IIIu), (IIIv), or (IIIw) 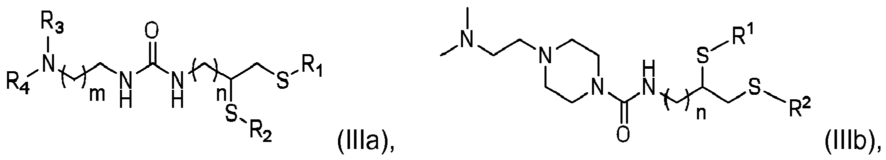 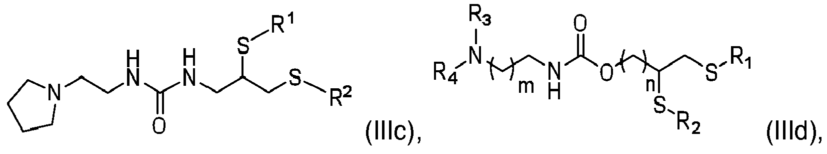 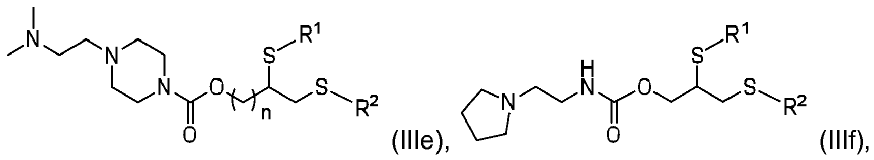 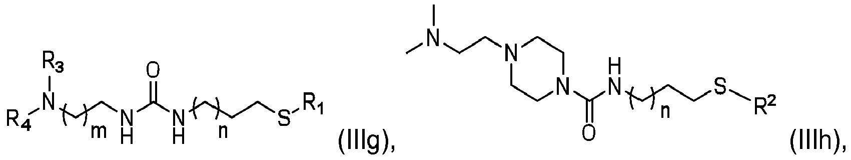 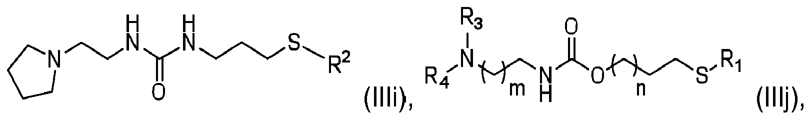 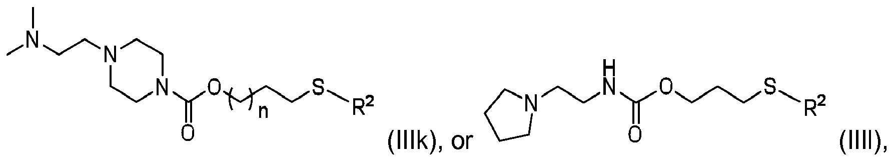 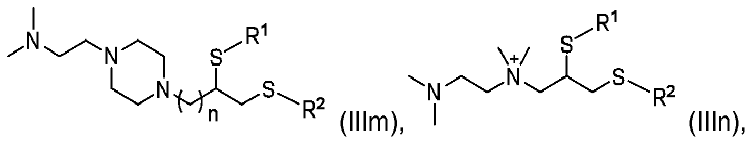 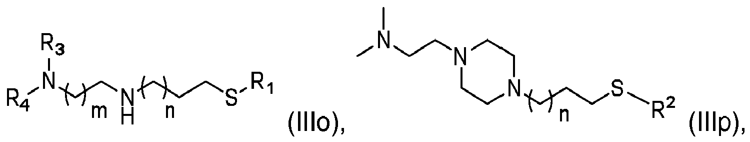 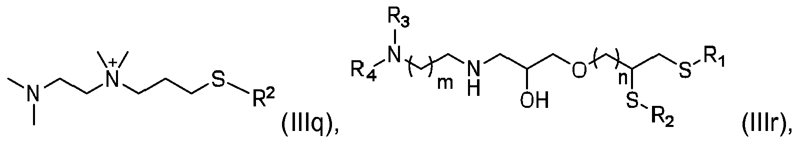 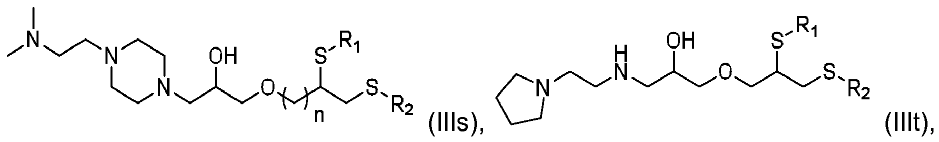 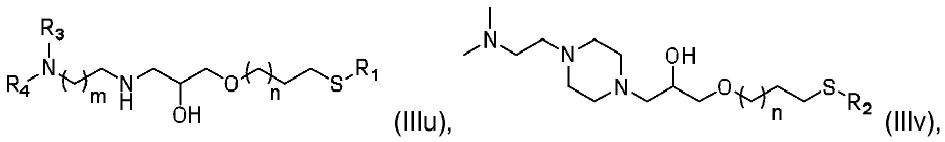 or 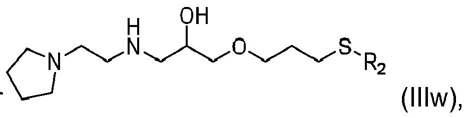 wherein R¹ and R² are the same C₁₁-C₁₂ alkyl,
R³ and R⁴ are the same C₁-C₂ alkyls,
m is an integer from 1 to 2, n is an integer from 1 to 3.

8. A lipid particle containing an amino lipid of one of the claims 1 to 7.

9. The lipid particle of claim 8, wherein said lipid particle is a liposome.

10. The lipid particle of claims 8 or 9, further containing a non-cationic lipid, a sterol and/or a bioactive agent, wherein the bioactive agent is in particular selected from the group consisting of: a nucleic acid, an antineoplastic agent, an antibiotic, an immunomodulator, an anti-inflammatory agent, an agent acting on the central nervous system, a polypeptide or a polypeptoid.

11. Use of a lipid particle of claims 8 to 10 for delivering a bioactive agent into a cell.

12. Use of a lipid particle of claims 8 to 10 as a medicament, in particular as a medicament in the treatment of a viral infection, a liver disease or disorder, or cancer.

13. A method for synthesizing an amino lipid of claims 1 and 4 to 7 comprising the step of:
performing a reaction of alkynes or alkenes of general formula (VI) 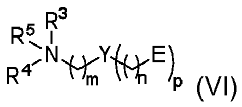 wherein E is either CH=CH₂ or C=CH,
with the compounds of the general formula HS-R¹ and HS-R² to yield a compound of general formula (VII) 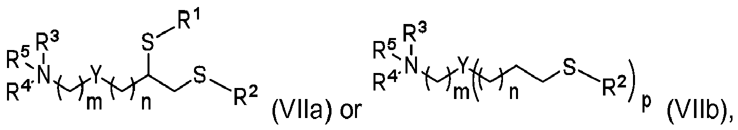 wherein R¹ and R² are the same or different and independently C₆-C₂₄ alkyl, C₆-C₂₄ alkenyl, C₆-C₂₄ alkynyl, or C₆-C₂₄ acyl, which is optionally substituted with a C₁-C₆ hydrocarbyl group,
Y is either 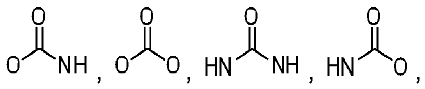 NH, NR⁷, ⁺NR⁶R⁷₂, 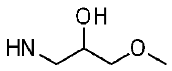 or a heterocycle of the formula 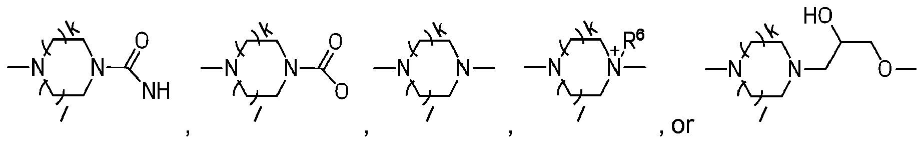 wherein k and I are integers from 0 or wherein k and I are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl is optionally substituted with a C₁-C₆ hydrocarbyl group, or R³ and R⁴ optionally join to form an optionally substituted heterocyclic ring of 3 to 10 atoms and 0 to 6 heteroatoms selected from a group consisting of nitrogen, sulfur and oxygen, or R³ and R⁴ are the same or different alkyl amines;
R⁵ and R⁶ are either absent or are hydrogen or C₁-C₁₂ alkyl to provide a quaternary amine,
R⁷ is either hydrogen or C₁-C₁₂ alkyl,
m is an integer from 1 to 12, n is an integer from 1 to 12, and p is an integer from 1 to 3, wherein preferably, Y = N for p = 2 or 3.

14. The method of claim 13, wherein the reaction of alkynes or alkenes of general formula (VI) with the compounds of the general formula HS-R¹ and HS-R² is performed under UV-irradiation or using a radical initiator.

15. The method of claim 12 or 14, wherein the product of the reaction is a compound according to formula (VIIb).

16. The method of claims 13 to 15, further comprising the step of oxidation of the thioethers of general formula (VIIa) or (VIIb) into sulfoxide (S=O) and/or sulfone (S(=O)₂) using an oxidation agent to synthesize a sulfoxide or sulfone group containing amino lipid of claim 1 or 2.

17. The method of claim 13 or 16, further comprising the steps of
a) Performing a reaction of alkynes or alkenes of the general formula (IVa), (IVb), (IVc), or (IVd) 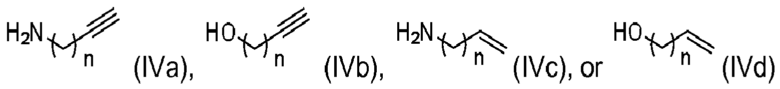 wherein n is an integer from 1 to 12,
with the compound 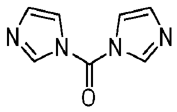 to yield a compound of the general formula (Va), (Vb), (Vc), or (Vd) 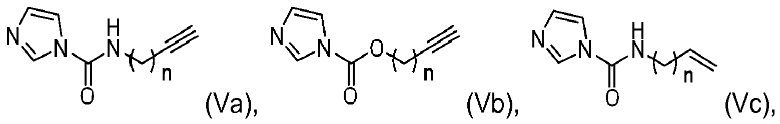 or 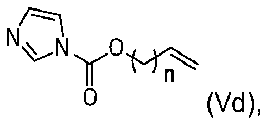 and/or
b) Performing a reaction of alkynes or alkenes of the general formula (Va), (Vb), (Vc), or (Vd) 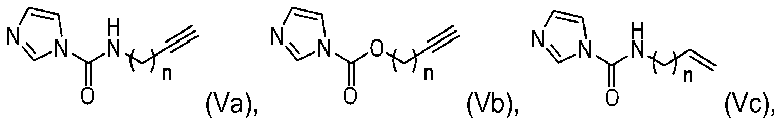 or 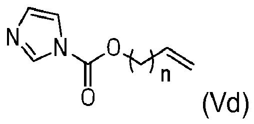 with an amine of the general formula (R³R⁴R⁵N)(CH₂)ₘZ, wherein m is an integer from 1 to 12, Z is OH, NH₂, NH, or a secondary heterocyclic amine of the formula 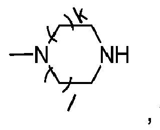 wherein k and I are integers from 0 to 2,
to yield a compound of the general formula (VI'a) or (VI'b) 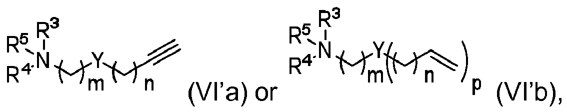 wherein R¹ and R² are the same or different and independently C₆-C₂₄ alkyl, C₆-C₂₄ alkenyl, C₆-C₂₄ alkynyl, or C₆-C₂₄ acyl, which is optionally substituted with a C₁-C₆ hydrocarbyl group,
Y is either 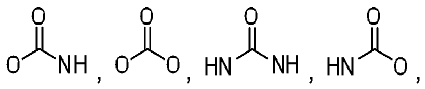 or heterocycles of the formula 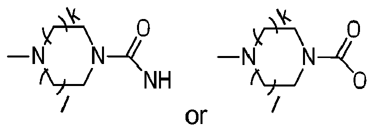 or wherein k and I are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl is optionally substituted with a C₁-C₆ hydrocarbyl group, or R³ and R⁴ optionally join to form an optionally substituted heterocyclic ring of 3 to 10 atoms and 0 to 6 heteroatoms selected from the group consisting of nitrogen, sulfur and oxygen, or R³ and R⁴ are the same or different alkyl amines;
R⁵ is either absent or is hydrogen or C₁-C₁₂ alkyl to provide a quaternary amine,
m is an integer from 1 to 12, n is an integer from 1 to 12, and p is an integer from 1 to 3, wherein preferably, Y = N for p = 2 or 3.

18. The method of claim 13 or 16, further comprising the step of Performing a reaction of alkynes or alkenes of the general formula (IVe), (IVf), (IVg), or (IVh) 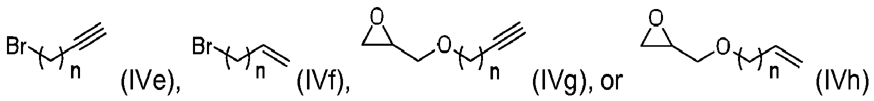 wherein n is an integer from 1 to 12,
with an amine of the general formula (R³R⁴R⁵N)(CH₂)ₘZ, wherein m is an integer from 1 to 12, and Z is NH₂, NH, N or a secondary heterocyclic amine of the formula 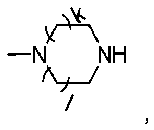 wherein k and I are integers from 0 to 2,
to yield a compound of the general formula (VI"a) or (VI"b) 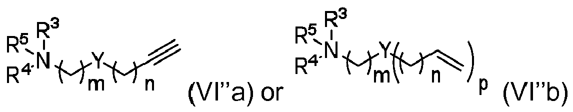 wherein R¹ and R² are the same or different and independently C₆-C₂₄ alkyl, C₆-C₂₄ alkenyl, C₆-C₂₄ alkynyl, or C₆-C₂₄ acyl, which is optionally substituted with a C₁-C₆ hydrocarbyl group,
Y is either NH, NR⁷, ⁺NR⁶R⁷, 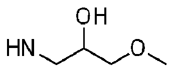 or heterocycles of the formula 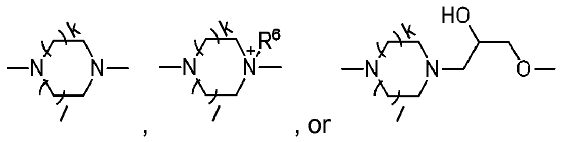 wherein k and I are integers from 0 to 2,
R³ and R⁴ are either the same or different and independently C₁-C₁₂ alkyl, C₁-C₁₂ alkenyl, or C₁-C₁₂ alkynyl, wherein alkyl, alkenyl or alkynyl is optionally substituted with a C₁-C₆ hydrocarbyl group, or R³ and R⁴ optionally join to form an optionally substituted heterocyclic ring of 3 to 10 atoms and 0 to 6 heteroatoms chosen from nitrogen, sulfur and oxygen,
R⁵ and R⁶ are either absent or are hydrogen or C₁-C₁₂ alkyls to provide a quaternary amine,
R⁷ is either hydrogen or C₁-C₁₂ alkyl,
m is an integer from 1 to 12, n is an integer from 1 to 12, and p is an integer from 1 to 3, wherein preferably, Y = N for p = 2 or 3.
